(19) Europäisches Patentamt
European Patent Office
Office européen des brevets

(11) **EP 3 150 643 B1**

(12) **EUROPEAN PATENT SPECIFICATION**

(45) Date of publication and mention of the grant of the patent:
**27.12.2017 Bulletin 2017/52**

(21) Application number: **15799081.3**

(22) Date of filing: **28.05.2015**

(51) Int Cl.:
***C08F 20/02*** *(2006.01)* ***C08F 220/02*** *(2006.01)*
***C09B 11/00*** *(2006.01)* ***C09B 11/20*** *(2006.01)*
***C09B 67/20*** *(2006.01)* ***C09B 69/10*** *(2006.01)*
***G02B 5/20*** *(2006.01)* ***C09B 11/14*** *(2006.01)*
***C09B 69/06*** *(2006.01)*

(86) International application number:
**PCT/JP2015/065333**

(87) International publication number:
**WO 2015/182680 (03.12.2015 Gazette 2015/48)**

(54) **TRIPHENYLMETHANE-BASED COLORED COMPOSITION**

TRIPHENYLMETHANBASIERTE GEFÄRBTE ZUSAMMENSETZUNG

COMPOSITION COLORÉE À BASE DE TRIPHENYLMÉTHANE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.05.2014 JP 2014113196**
**27.01.2015 JP 2015012957**

(43) Date of publication of application:
**05.04.2017 Bulletin 2017/14**

(73) Proprietor: **Wako Pure Chemical Industries, Ltd.**
**Osaka-shi**
**Osaka 540-8605 (JP)**

(72) Inventors:
- **HORIE, Tomoaki**
**Kawagoe-shi**
**Saitama 350-1101 (JP)**
- **YANABA, Kosuke**
**Kawagoe-shi**
**Saitama 350-1101 (JP)**
- **HISHINUMA, Yusuke**
**Kawagoe-shi**
**Saitama 350-1101 (JP)**

(74) Representative: **Hinkelmann, Klaus**
**Patentanwaltskanzlei Hinkelmann**
**Lyonel-Feininger-Strasse 28**
**80807 München (DE)**

(56) References cited:
**EP-A1- 0 912 639** **WO-A1-2012/005203**
**WO-A1-2013/176383** **WO-A1-2015/045322**
**WO-A1-2015/093501** **WO-A1-2015/098999**
**WO-A1-2015/115282** **JP-A- 2003 206 415**
**JP-A- 2012 072 205** **JP-A- 2012 073 291**
**JP-A- 2013 163 804** **JP-A- 2014 240 939**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 150 643 B1

## Description

TECHNICAL FIELD

The present invention relates to a triphenylmethane-based colored compound which is used in an application of formation of a colored pixel such as a color filter, or in applications of printing ink, inkjet ink, paint, and the like; a polymer having a monomer unit derived from the compound; and a colored composition comprising the polymer.

BACKGROUND ART

As a method for forming a colored pixel in manufacturing a color filter of a liquid crystal display element, a solid-state image sensing element, or the like; a dyeing method or a dye-dispersion method employing a dye for a colorant, a pigment-dispersion method using a pigment, an electrodeposition method, a printing method, or the like, has been known. In recent years, as characteristics of the color filter, enhancement of brightness and contrast is particularly required. According to the pigment-dispersion method using the pigment, because of higher heat resistance and light resistance as compared with the dye, the colored pixel having less deterioration at heating process in manufacturing a panel, and also having high long-term reliability can be obtained. Therefore, at present, the pigment-dispersion method has become the mainstream. However, when the pigment was used, because the pigment itself has relatively large particle size, there was a problem that contrast may be decreased by light scattering. Although an attempt has also been made to micronize the pigment, there is a limit in micronization, and it has also been a problem to secure dispersion stability of the micronized pigment.

On the other hand, as a method to resolve these problems, a method for forming the colored pixel using a dye has been studied at present. When the dye is used, contrast is enhanced because light scattering is suppressed. However, because the dye has lower heat resistance as compared with the pigment, and has sublimation depending on the type, there were problems, such as reduction in brightness, fading and hue change. Therefore, in the method using the dye, it has been required to resolve these problems. Regarding the dye having heat resistance, various studies have been made. For example, in WO2010/123071 or WO2013/108591, a triarylmethane-based coloring compound having a sulfonyl imide-based counter anion has been reported.

WO 2013/176383 A1 deals with triarylmethane dyes suitable for colour-filters thereby showing high heat resistance; the polymerized diphenyl-naphthyl-methane dyes of JP 2013-163804 A are also used in colour-filters showing high heat resistance.

CITATION LIST

PATENT LITERATURE

[PATENT LITERATURE 1] WO2010/123071
[PATENT LITERATURE 2] WO2013/108591
[PATENT LITERATURE 3] WO2013/176383
[PATENT LITERATURE 4] JP2013-163804

SUMMARY OF INVENTION

TECHNICAL PROBLEM

As a result of studies on a colored resin composition using the conventional triarylmethane-based coloring compound, heat resistance within a practical range has not been obtained. Therefore, it is an object of the present invention to provide a colored composition having higher heat resistance as compared with a conventional colored composition.

SOLUTION TO PROBLEM

In view of the above-situation, the present inventors, as a result of intensive study, have discovered that a colored composition having excellent heat resistance and less dye elution is obtained, by using the triphenylmethane-based compound which has a cationic triphenylmethane derivative having a specific anion, as a counter anion, and an ethylenically unsaturated bond, or a polymer having a monomer unit derived from the compound, as a dye, and have

completed the present invention.

That is, the present invention relates to "a compound represented by the following general formula (1) (hereinafter, it may be abbreviated as the compound of the present invention):

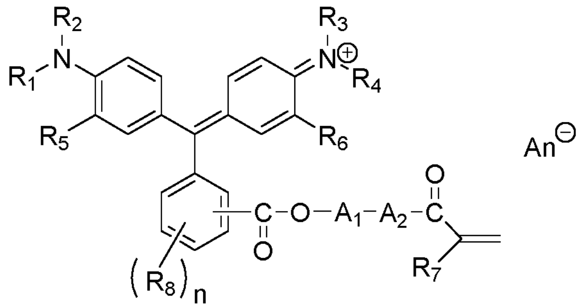

(1)

(wherein $R_1$ to $R_4$ each independently represent a hydrogen atom, an alkyl group having 1 to 30 carbon atoms, a hydroxyalkyl group having 1 to 6 carbon atoms, a sulfoalkyl group having 1 to 6 carbon atoms, a carboxyalkyl group having 2 to 7 carbon atoms, a cyanoalkyl group having 2 to 7 carbon atoms, an alkoxyalkyl group having 2 to 6 carbon atoms, a halogenated alkyl group having 1 to 6 carbon atoms, or a phenyl group, a naphthyl group or a benzyl group, having a substituent or not having a substituent; $R_5$ to $R_7$ each independently represent a hydrogen atom or a methyl group; n pieces of $R_8$ each independently represent a halogen atom, an alkyl group having 1 to 21 carbon atoms, an aryl group having 6 to 10 carbon atoms, a hydroxy group, a nitro group, a sulfo group, or an alkoxy group having 1 to 3 carbon atoms; and n represents an integer of 0 to 4. $A_1$ represents an alkylene group having 1 to 21 carbon atoms, which has at least one group selected from -O-, -OCO-, -COO- and an arylene group in the chain, an alkylene group having 1 to 21 carbon atoms, which has at least one group selected from -O-, -OCO-, -COO- and an arylene group in the chain, and also has a hydroxy group as a substituent, an alkylene group having 1 to 21 carbon atoms which has a hydroxy group as a substituent, or an alkylene group having 1 to 21 carbon atoms. $A_2$ represents -NH- or -O-. $An^-$ represents an anion containing an aryl group having an electron-withdrawing substituent, a sulfonyl group having an electron-withdrawing substituent, or a halogenated alkyl group.)",

"a polymer having a monomer unit derived from the compound represented by the general formula (1) (hereinafter, it may be abbreviated as the polymer of the present invention)",

"a colored composition comprising the compound or the polymer", and

"a colored composition for a color filter comprising the compound or the polymer".

## ADVANTAGEOUS EFFECTS OF INVENTION

When the compound or the polymer of the present invention is used as the coloring agent, even if heating is carried out at 230°C for 30 minutes, fading caused by heating is less, and high heat resistance effect is exerted. That is, the colored composition containing the compound or the polymer of the present invention has superior heat resistance effect as compared with the conventional colored composition, thus it is capable of forming a superior colored cured film. Therefore, the colored composition of the present invention can be used in an application of formation of a colored pixel such as a color filter used in a liquid crystal display (LCD) or a solid-state imaging element (CCD, CMOS, or the like), or in applications of printing ink, inkjet ink, paint, and the like; and particularly, it can be suitably used for the color filter of the liquid crystal display. Still more, the colored composition of the present invention can also be used as a colored resin molded articles by molding to a sheet, a film, a bottle, a cup, or the like, using a conventionally known molding method. Accordingly, it can also be used in applications of spectacles, contact lens, color contact lens, or the like; and it can be used in similar applications also by making a multi-layered structure with a known resin. In addition, it can also be used in applications of, for example, an optical film, a hair coloring agent, a labeling material for a compound or a biomaterial, a material of an organic solar battery, or the like.

In addition, when the compound or the polymer of the present invention is used by mixing into a resist material as a coloring agent, it exerts the effect that dye (coloring agent) elution is less. Therefore, as compared with the conventional colored composition, it is capable of providing an excellent colored composition not having problems of deterioration of color concentration, color mixture, and the like.

DESCRIPTION OF EMBODIMENTS

[Anion containing the aryl group having the electron-withdrawing substituent, the sulfonyl group having the electron-withdrawing substituent, or the halogenated alkyl group]

An anion moiety in the anion containing the aryl group having the electron-withdrawing substituent, the sulfonyl group having the electron-withdrawing substituent, or the halogenated alkyl group, represented by $An^-$ of the general formula (1) (hereinafter, it may be abbreviated as the anion of the present invention), includes, for example, a sulfonate anion, a nitrogen anion ($N^-$), a quaternary boron anion, a nitrate ion, a phosphate ion, and the like, and a sulfonate anion, a nitrogen anion and a quaternary boron anion are preferable, and a quaternary boron anion is more preferable.

The electron-withdrawing substituent in the aryl group having the electron-withdrawing substituent, or the sulfonyl group having the electron-withdrawing substituent, in the anion of the present invention, includes, for example, a halogenated alkyl group having 1 to 3 carbon atoms, a halogeno group, a nitro group, and the like. Among them, a halogenated alkyl group having 1 to 3 carbon atoms, and a halogeno group are preferable, and a halogeno group is particularly preferable.

The halogenated alkyl group having 1 to 3 carbon atoms, as the electron-withdrawing substituent, includes, for example, a chloroalkyl group such as a chloromethyl group, a trichloromethyl group, a 2-chloroethyl group, a 2,2,2-trichloroethyl group, a pentachloroethyl group, a 2-chloropropyl group, a 3-chloropropyl group, a 2-chloro-2-propyl group and a heptachloropropyl group; a bromoalkyl group such as a bromomethyl group, a tribromomethyl group, a 2-bromoethyl group, a 2,2,2-tribromoethyl group, a pentabromoethyl group, a 2-bromopropyl group, a 3-bromopropyl group, a 2-bromo-2-propyl group and a heptabromopropyl group; an iodoalkyl group such as an iodomethyl group, a triiodomethyl group, a 2-iodoethyl group, a 2,2,2-triiodoethyl group, a pentaiodoethyl group, a 2-iodopropyl group, a 3-iodopropyl group, a 2-iodo-2-propyl group and a heptaiodopropyl group; and a fluoroalkyl group such as a fluoromethyl group, a trifluoromethyl group, a 2-fluoroethyl group, a 2,2,2-trifluoroethyl group, a 1,1,2,2-tetrafluoroethyl group, a pentafluoroethyl group, a 3-fluoropropyl group, a 3,3,3-trifluoropropyl group, a 2,2,3,3-tetrafluoropropyl group and a heptafluoropropyl group. Among them, a perhalogeno alkyl group such as a trichloromethyl group, a pentachloroethyl group, a heptachloropropyl group, a tribromomethyl group, a pentabromoethyl group, a heptabromopropyl group, a triiodomethyl group, a pentaiodoethyl group, a heptaiodopropyl group, a trifluoromethyl group, a pentafluoroethyl group and a heptafluoropropyl group, is preferable, and a perfluoroalkyl group such as a trifluoromethyl group, a pentafluoroethyl group and a heptafluoropropyl group, is more preferable, and a trifluoromethyl group is particularly preferable.

The halogeno group as the electron-withdrawing substituent includes a fluoro group, a chloro group, a bromo group and an iodo group, and a fluoro group is preferable.

As the electron-withdrawing substituent in the aryl group having the electron-withdrawing substituent in the anion of the present invention, among the above-described specific examples, the one having strong electron-withdrawing ability is preferable, and a trifluoromethyl group, a fluoro group and a nitro group are preferable, and a fluoro group is more preferable.

As the electron-withdrawing substituent in the sulfonyl group having the electron-withdrawing substituent in the anion of the present invention, among the above-described specific examples, a trifluoromethyl group, a pentafluoroethyl group, a heptafluoropropyl group and a fluoro group are preferable.

The aryl group in the aryl group having the electron-withdrawing substituent in the anion of the present invention includes, for example, a phenyl group, a naphthyl group, and the like, and a phenyl group is preferable.

Specific examples of the aryl group having the electron-withdrawing substituent, in the anion of the present invention, include, for example, those represented by the following general formulae (11) and (12).

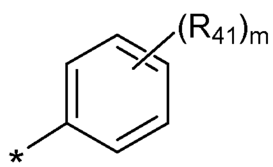

(11)

(wherein m represents an integer of 1 to 5; m pieces of $R_{41}$ each independently represent a halogenated alkyl group having 1 to 3 carbon atoms, a halogen atom or a nitro group; and * represents an atomic bonding.)

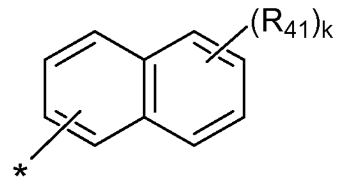

(12)

(wherein k represents an integer of 1 to 7; $R_{41}$ and * are the same as described above; and k pieces of $R_{41}$ may be the same or different.)

As for m, it is usually an integer of 1 to 5, and, in the case where $R_{41}$ is a halogen atom, 2 to 5 is preferable, and 3 to 5 is more preferable, and 5 is still more preferable. In the case where $R_{41}$ is a nitro group, 1 to 3 is preferable, and 1 is more preferable. In the case where $R_{41}$ is a halogenated alkyl group, 1 to 5 is preferable, and 1 to 3 is more preferable.

As for k, it is usually an integer of 1 to 7, and, in the case where $R_{41}$ is a halogen atom, 2 to 7 is preferable. In the case where $R_{41}$ is a nitro group, 1 to 3 is preferable, and 1 is more preferable. In the case where $R_{41}$ is a halogenated alkyl group, 1 to 7 is preferable, and 1 to 3 is more preferable.

The halogenated alkyl group having 1 to 3 carbon atoms, in $R_{41}$ of the general formula (11) and the general formula (12), includes the same one as the halogenated alkyl group having 1 to 3 carbon atoms, as the electron-withdrawing substituent in the anion of the present invention, and the preferable ones are also the same.

The halogen atom in $R_{41}$ of the general formula (11) and the general formula (12) includes a fluorine atom, a chlorine atom, a bromine atom, an iodine atom, and the like, and among them, a fluorine atom is preferable.

Preferable specific examples of $R_{41}$ in the general formula (11) and the general formula (12) are the same as the preferable ones of the electron-withdrawing substituent in the aryl group having the electron-withdrawing substituent.

The group represented by the general formula (11) specifically includes, for example, a trifluoromethylphenyl group, a di(trifluoromethyl)phenyl group, a tri(trifluoromethyl)phenyl group, a monofluorophenyl group, a difluorophenyl group, a trifluorophenyl group, a perfluorophenyl group, a monochlorophenyl group, a dichlorophenyl group, a trichlorophenyl group, a perchlorophenyl group, a monobromophenyl group, a dibromophenyl group, a tribromophenyl group, a perbromophenyl group, a monoiodophenyl group, a diiodophenyl group, a triiodophenyl group, a periodophenyl group, a nitrophenyl group, a dinitrophenyl group, a trinitrophenyl group, and the like, and a difluorophenyl group, a trifluorophenyl group and a perfluorophenyl group are preferable, and a perfluorophenyl group is more preferable.

The group represented by the general formula (12) specifically includes, for example, a trifluoromethylnaphthyl group, a di(trifluoromethyl)naphthyl group, a tri(trifluoromethyl)naphthyl group, a monofluoronaphthyl group, a difluoronaphthyl group, a trifluoronaphthyl group, a perfluoronaphthyl group, a monochloronaphthyl group, a dichloronaphthyl group, a trichloronaphthyl group, a perchloronaphthyl group, a monobromonaphthyl group, a dibromonaphthyl group, a tribromonaphthyl group, a perbromonaphthyl group, a monoiodonaphthyl group, a diiodonaphthyl group, a triiodonaphthyl group, a periodonaphthyl group, a nitronaphthyl group, a dinitronaphthyl group, a trinitronaphthyl group, and the like.

As the aryl group having the electron-withdrawing substituent in the anion of the present invention, among the above-described specific examples, the group represented by the general formula (11) is preferable, and specifically, a trifluoromethylphenyl group, a nitrophenyl group, a dinitrophenyl group, a trinitrophenyl group, a monofluorophenyl group, a difluorophenyl group, a trifluorophenyl group and a perfluorophenyl group are preferable, and a difluorophenyl group, a trifluorophenyl group, a nitrophenyl group and a perfluorophenyl group are more preferable, and a perfluorophenyl group is further particularly preferable.

The sulfonyl group having the electron-withdrawing substituent in the anion of the present invention includes, for example, $-SO_2-CF_3$, $-SO_2-C_2F_5$, $-SO_2-C_3F_7$, $-SO_2-F$, $-SO_2-Cl$, $-SO_2-Br$, $-SO_2-I$, and the like.

The halogenated alkyl group in the anion of the present invention includes a halogenated alkyl group having 1 to 3 carbon atoms, and among them, a perhalogenated alkyl group is preferable, specifically including, for example, a trifluoromethyl group, a pentafluoroethyl group, a heptafluoropropyl group, a trichloromethyl group, a pentachloroethyl group, a heptachloropropyl group, a tribromomethyl group, a pentabromoethyl group, a heptabromopropyl group, a triiodomethyl group, a pentaiodoethyl group, a heptaiodopropyl group, and the like, and a trifluoromethyl group, a pentafluoroethyl group and a heptafluoropropyl group are preferable.

The anion containing the aryl group having the electron-withdrawing substituent, the sulfonyl group having the electron-withdrawing substituent, or the halogenated alkyl group, pertaining to the present invention, specifically includes, for example, those represented by the following general formulae (13) to (18).

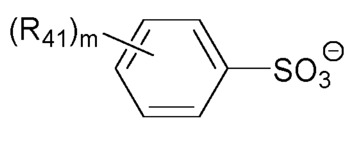

(13)

(wherein $R_{41}$ and m are the same as described above; and m pieces of $R_{41}$ may be the same or different.)

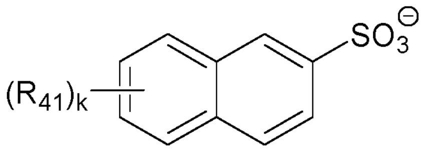

(14)

(wherein $R_{41}$ and k are the same as described above; and k pieces of $R_{41}$ may be the same or different.)

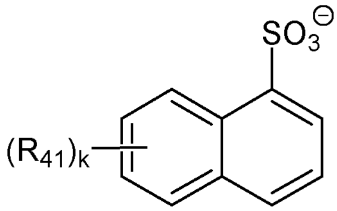

(15)

(wherein $R_{41}$ and k are the same as described above; and k pieces of $R_{41}$ may be the same or different.)

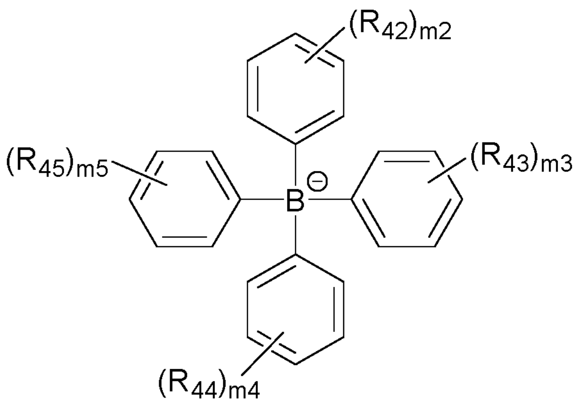

(16)

(wherein $R_{42}$ to $R_{45}$ each independently represent a halogenated alkyl group having 1 to 3 carbon atoms, a halogen atom, or a nitro group; $m_2$ to $m_5$ each independently represent an integer of 1 to 5; and $m_2$ pieces of $R_{42}$, $m_3$ pieces of $R_{43}$, $m_4$ pieces of $R_{44}$, and $m_5$ pieces of $R_{45}$ each may be the same or different.)

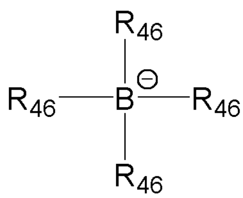

(17)

(wherein $R_{46}$ each independently represent a halogenated alkyl group having 1 to 3 carbon atoms, or a halogen atom; provided that at least one of four $R_{46}$ represents a halogenated alkyl group having 1 to 3 carbon atoms.)

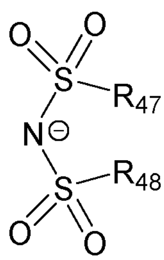

(18)

(wherein $R_{47}$ and $R_{48}$ each independently represent a halogenated alkyl group having 1 to 3 carbon atoms, or a halogen atom; and $R_{47}$ together with $R_{48}$ may form a halogenated alkylene group having 2 to 3 carbon atoms.)

Combinations of $R_{41}$ and m in the general formula (13) include, for example, those described in the following table. It should be noted that the m pieces of $R_{41}$ are each independent, and it is preferable that they are all the same.

| $R_{41}$ | m |
|---|---|
| trifluoromethyl group ($-CF_3$) | 1 to 3 |
| pentafluoroethyl group ($-C_2F_5$) | 1 to 3 |
| heptafluoropropyl group ($-C_3F_7$) | 1 to 3 |
| nitro group | 1 to 3 |
| fluorine atom | 1 to 5 |
| chlorine atom | 1 to 5 |
| bromine atom | 1 to 5 |
| iodine atom | 1 to 5 |

Preferable specific examples of the anion represented by the general formula (13) include, for example, the following ones.

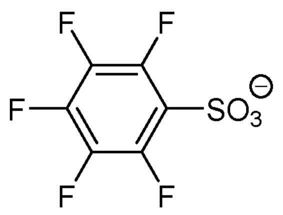
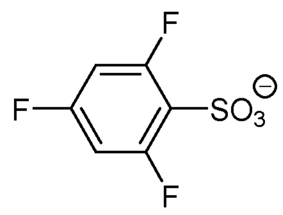
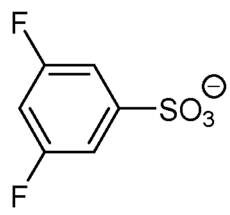
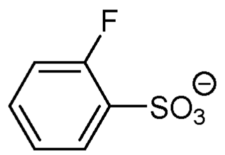
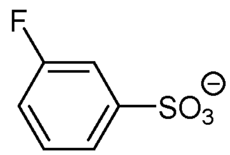
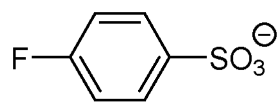
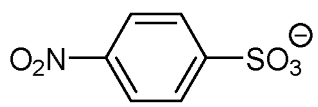
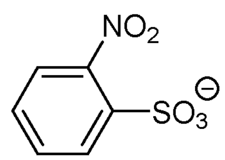
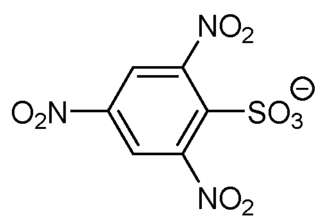
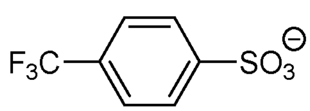
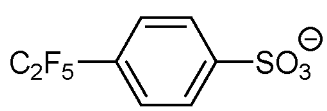
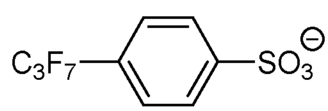

Combinations of $R_{41}$ and k in the general formulae (14) and (15) include, for example, those described in the following table. It should be noted that the k pieces of $R_{41}$ are each independent, and it is preferable that they are all the same.

| $R_{41}$ | k |
|---|---|
| trifluoromethyl group ($-CF_3$) | 1 to 3 |
| pentafluoroethyl group ($-C_2F_5$) | 1 to 3 |

(continued)

| $R_{41}$ | k |
|---|---|
| heptafluoropropyl group ($-C_3F_7$) | 1 to 3 |
| nitro group | 1 to 3 |
| fluorine atom | 1 to 7 |
| chlorine atom | 1 to 7 |
| bromine atom | 1 to 7 |
| iodine atom | 1 to 7 |

Preferable specific examples of the anions represented by the general formulae (14) and (15) include, for example, the following ones.

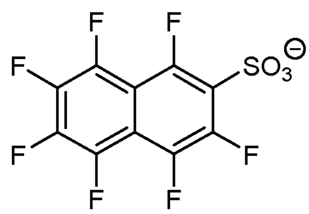
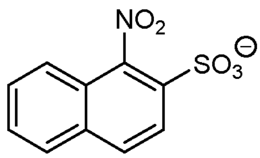
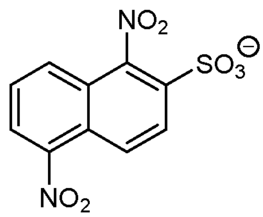

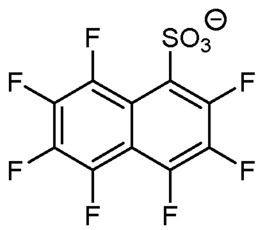
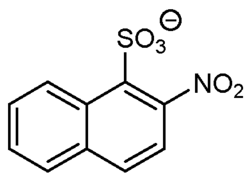
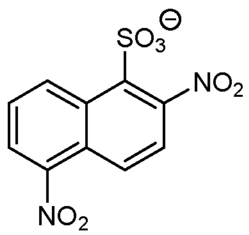

The halogenated alkyl group having 1 to 3 carbon atoms, in $R_{42}$ to $R_{45}$ of the general formula (16), includes the same one as the halogenated alkyl group having 1 to 3 carbon atoms, as the electron-withdrawing substituent in the anion of the present invention, and the preferable ones are also the same.

The halogen atom in $R_{42}$ to $R_{45}$ of the general formula (16) includes a fluorine atom, a chlorine atom, a bromine atom, an iodine atom, and the like, and among them, a fluorine atom is preferable.

Combinations of $R_{42}$ to $R_{45}$ and $m_2$ to $m_5$ in the general formula (16) include, for example, those described in the following table.

| $R_{42}$ | $m_2$ | $R_{43}$ | $m_3$ | $R_{44}$ | $m_4$ | $R_{45}$ | $m_5$ |
|---|---|---|---|---|---|---|---|
| $-CF_3$ | 1 to 3 | $-CF_3$ | 1 to 3 | $-CF_3$ | 1 to 3 | $-CF_3$ | 1 to 3 |
| $-C_2F_5$ | 1 to 3 | $-C_2F_5$ | 1 to 3 | $-C_2F_5$ | 1 to 3 | $-C_2F_5$ | 1 to 3 |
| $-C_3F_7$ | 1 to 3 | $-C_3F_7$ | 1 to 3 | $-C_3F_7$ | 1 to 3 | $-C_3F_7$ | 1 to 3 |
| nitro group | 1 to 3 | nitro group | 1 to 3 | nitro group | 1 to 3 | nitro group | 1 to 3 |
| fluorine | 1 to 5 | fluorine | 1 to 5 | fluorine | 1 to 5 | fluorine | 1 to 5 |
| chlorine | 1 to 5 | chlorine | 1 to 5 | chlorine | 1 to 5 | chlorine | 1 to 5 |
| bromine | 1 to 5 | bromine | 1 to 5 | bromine | 1 to 5 | bromine | 1 to 5 |
| iodine | 1 to 5 | iodine | 1 to 5 | iodine | 1 to 5 | iodine | 1 to 5 |
| nitro group | 1 to 3 | fluorine | 1 to 5 | fluorine | 1 to 5 | fluorine | 1 to 5 |
| nitro group | 1 to 3 | nitro group | 1 to 3 | fluorine | 1 to 5 | fluorine | 1 to 5 |

(continued)

| $R_{42}$ | $m_2$ | $R_{43}$ | $m_3$ | $R_{44}$ | $m_4$ | $R_{45}$ | $m_5$ |
|---|---|---|---|---|---|---|---|
| nitro group | 1 to 3 | nitro group | 1 to 3 | nitro group | 1 to 3 | fluorine | 1 to 5 |

Preferable specific examples of the anion represented by the general formula (16) include, for example, the following ones.

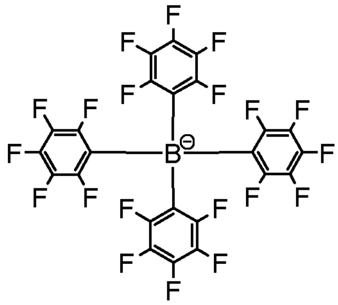
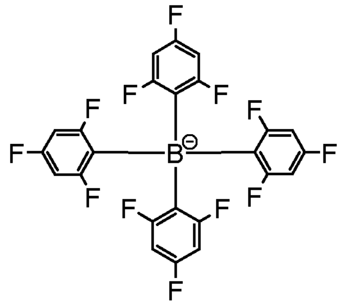
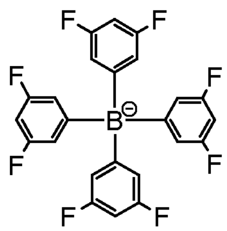
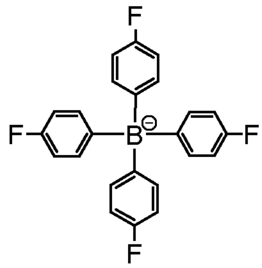
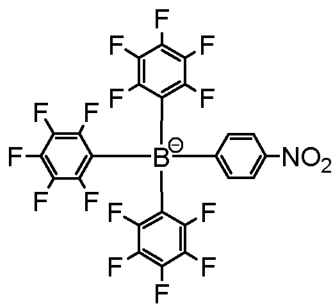
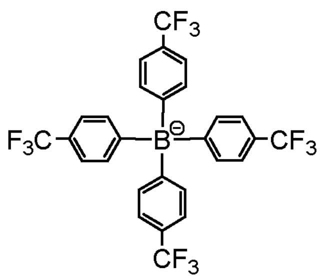
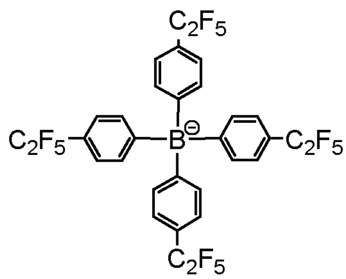

Among the above-described specific examples, the following ones are more preferable.

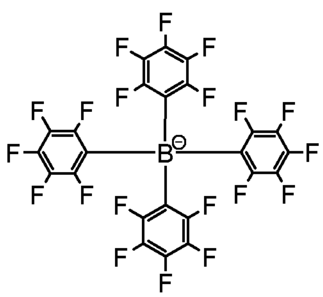
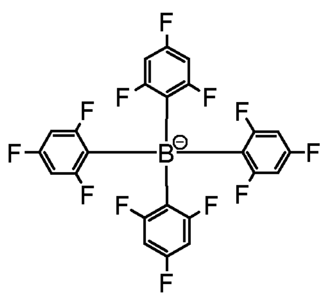
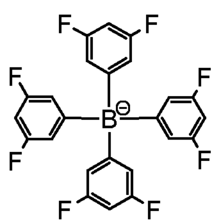

Among the above-described specific examples, the following one is particularly preferable.

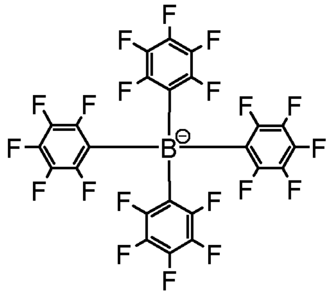

The halogenated alkyl group having 1 to 3 carbon atoms, in $R_{46}$ of the general formula (17), includes the same one as the halogenated alkyl group having 1 to 3 carbon atoms, as the electron-withdrawing substituent in the anion of the present invention, and the preferable ones are also the same.

The halogen atom in $R_{46}$ of the general formula (17) includes a fluorine atom, a chlorine atom, a bromine atom, an iodine atom, and the like, and among them, a fluorine atom is preferable.

Preferable specific examples of the anion represented by the general formula (17) include, for example, $CF_3BF_3^-$, $C_2F_5BF_3^-$, $C_3F_7BF_3^-$, $(CF_3)_4B^-$, $(C_2F_5)_4B^-$, $(C_3F_7)_4B^-$, and the like.

The halogenated alkyl group having 1 to 3 carbon atoms, in $R_{47}$ and $R_{48}$ of the general formula (18), includes the same one as the halogenated alkyl group having 1 to 3 carbon atoms, as the electron-withdrawing substituent in the anion of the present invention, and the preferable ones are also the same.

The halogen atom in $R_{47}$ and $R_{48}$ of the general formula (18) includes a fluorine atom, a chlorine atom, a bromine atom, an iodine atom, and the like, and among them, a fluorine atom is preferable.

The halogenated alkylene group having 2 to 3 carbon atoms formed by $R_{47}$ together with $R_{48}$ of the general formula (18) includes, for example, a tetrafluoroethylene group, a hexafluoropropylene group, and the like, and a hexafluoropropylene group is preferable.

Preferable specific examples of the anion represented by the general formula (18) include, for example, the following ones.

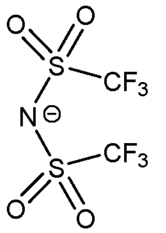 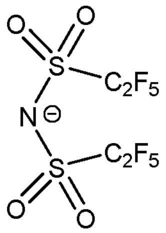 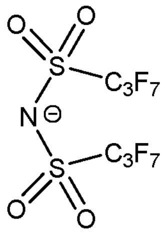 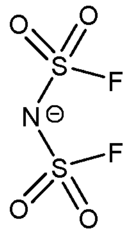 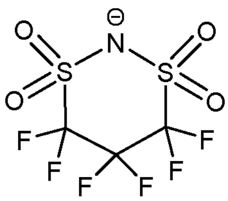

As the anion of the present invention, the one represented by the general formula (16), the general formula (17) or the general formula (18) is preferable, and the one represented by the general formula (16) or the general formula (18) is more preferable, and the one represented by the general formula (16) is particularly preferable.

As the anion of the present invention, among the above-described specific examples, the following ones are preferable.

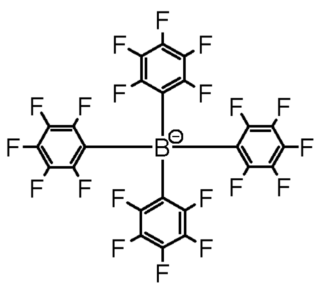 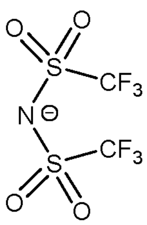 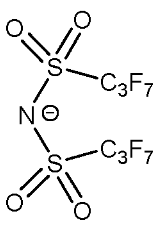 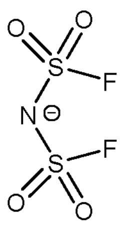 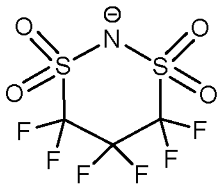

Among the above-described specific examples, the following one is more preferable.

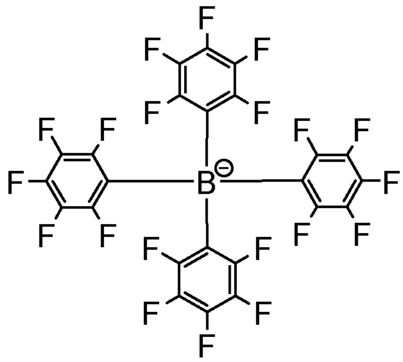

[Compound of the present invention]

The compound of the present invention is a compound represented by the general formula (1).

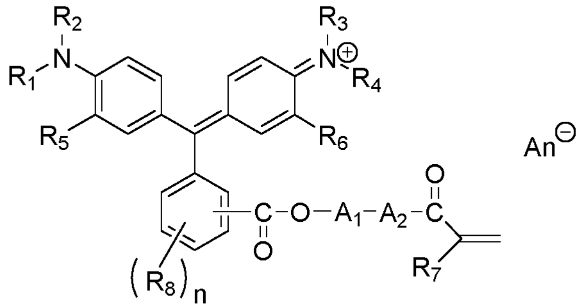

(1)

(wherein $R_1$ to $R_4$ each independently represent a hydrogen atom, an alkyl group having 1 to 30 carbon atoms, a hydroxyalkyl group having 1 to 6 carbon atoms, a sulfoalkyl group having 1 to 6 carbon atoms, a carboxyalkyl group having 2 to 7 carbon atoms, a cyanoalkyl group having 2 to 7 carbon atoms, an alkoxyalkyl group having 2 to 6 carbon atoms, a halogenated alkyl group having 1 to 6 carbon atoms, or a phenyl group, a naphthyl group or a benzyl group, having a substituent or not having a substituent; $R_5$ to $R_7$ each independently represent a hydrogen atom or a methyl group; n pieces of $R_8$ each independently represent a halogen atom, an alkyl group having 1 to 21 carbon atoms, an aryl group having 6 to 10 carbon atoms, a hydroxy group, a nitro group, a sulfo group, or an alkoxy group having 1 to 3 carbon atoms; and n represents an integer of 0 to 4. $A_1$ represents an alkylene group having 1 to 21 carbon atoms, which has at least one group selected from -O-, -OCO-, -COO- and an arylene group in the chain, an alkylene group having 1 to 21 carbon atoms, which has at least one group selected from -O-, -OCO-, -COO- and an arylene group in the chain, and also has a hydroxy group as a substituent, an alkylene group having 1 to 21 carbon atoms which has a hydroxy group as a substituent, or an alkylene group having 1 to 21 carbon atoms. $A_2$ represents -NH- or -O-. $An^-$ represents an anion containing an aryl group having an electron-withdrawing substituent, a sulfonyl group having an electron-withdrawing substituent, or a halogenated alkyl group.)

The alkyl group having 1 to 30 carbon atoms, in $R_1$ to $R_4$ of the general formula (1), may be any of the linear, branched, or cyclic one, and the one having 1 to 12 carbon atoms is preferable, and the one having 1 to 6 carbon atoms is more preferable, and the one having 1 to 3 carbon atoms is further preferable. Specifically it includes, for example, a methyl group, an ethyl group, a propyl group, an isopropyl group, a butyl group, a 1-methylpropyl group, an isobutyl group, a tert-butyl group, a pentyl group, a 1-ethylpropyl group, a 1-methylbutyl group, a cyclopentyl group, a hexyl group, a 1-methylpentyl group, a 1-ethylbutyl group, a cyclohexyl group, a 2-heptyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a henicosyl group, a docosyl group, a tricosyl group, a tetracosyl group, a pentacosyl group, a hexacosyl group, a heptacosyl group, an octacosyl group, a nonacosyl group, a triacontyl group, an isopentyl group, an isohexyl group, an isoheptyl group, an isooctyl group, an isononyl group, an isodecyl group, an isoundecyl group, an isododecyl group, an isotridecyl group, an isotetradecyl group, an isopentadecyl group, an isohexadecyl group, an isoheptadecyl group, an isooctadecyl group, an isononadecyl group, an isoicosyl group, an isohenicosyl group, an isodocosyl group, an isotricosyl group, an isotetracosyl group, an isopentacosyl group, an isohexacosyl group, an isoheptacosyl group, an isooctacosyl group, an isononacosyl group, an isotriacontyl group, a 1-methylhexyl group, a 2-ethylhexyl group, a 1-methylheptyl group, a 1-cyclohexylethyl group, a 1-ethylheptyl group, a 1-heptyloctyl group, a 2-methylcyclohexyl group, a 3-methylcyclohexyl

group, a 4-methylcyclohexyl group, a 2,6-dimethylcyclohexyl group, a 2,4-dimethylcyclohexyl group, a 3,5-dimethylcyclohexyl group, a 2,5-dimethylcyclohexyl group, a 2,3-dimethylcyclohexyl group, a 3,3,5-trimethylcyclohexyl group, a 4-tert-butylcyclohexyl group, a 1-adamantyl group, a 2-adamantyl group, and the like; a methyl group, an ethyl group, a propyl group, a isopropyl group, a butyl group, a pentyl group and a hexyl group are preferable; a methyl group, an ethyl group and a propyl group are more preferable; and a methyl group and a ethyl group are particularly preferable.

As the hydroxyalkyl group having 1 to 6 carbon atoms in $R_1$ to $R_4$ of the general formula (1), the one having 1 to 3 carbon atoms is preferable, specifically including, for example, a hydroxymethyl group, a hydroxyethyl group, a hydroxypropyl group, a hydroxybutyl group, a hydroxypentyl group, a hydroxyhexyl group, and the like.

As the sulfoalkyl group having 1 to 6 carbon atoms in $R_1$ to $R_4$ of the general formula (1), the one having 1 to 3 carbon atoms is preferable, specifically including, for example, a sulfomethyl group, a sulfoethyl group, a sulfopropyl group, a sulfobutyl group, a sulfopentyl group, a sulfohexyl group, and the like.

As the carboxyalkyl group having 2 to 7 carbon atoms in $R_1$ to $R_4$ of the general formula (1), the one having 3 to 6 carbon atoms is preferable, specifically including, for example, a carboxymethyl group, a carboxyethyl group, a carboxypropyl group, a carboxybutyl group, a carboxypentyl group, a carboxyhexyl group, and the like, and a carboxyethyl group is preferable.

As the cyanoalkyl group having 2 to 7 carbon atoms in $R_1$ to $R_4$ of the general formula (1), the one having 2 to 4 carbon atoms is preferable, specifically including, for example, a cyanomethyl group, a cyanoethyl group, a cyanopropyl group, a cyanobutyl group, a cyanopentyl group, a cyanohexyl group, and the like, and a cyanoethyl group is preferable.

As the alkoxyalkyl group having 2 to 6 carbon atoms in $R_1$ to $R_4$ of the general formula (1), the one having 3 to 5 carbon atoms is preferable, specifically including, for example, a methoxymethyl group, a methoxyethyl group, an ethoxymethyl group, an ethoxyethyl group, a propoxymethyl group, a propoxyethyl group, a butoxymethyl group, a butoxyethyl group, and the like.

As the halogenated alkyl group having 1 to 6 carbon atoms in $R_1$ to $R_4$ of the general formula (1), the one having 1 to 3 carbon atoms is preferable, specifically including, for example, a trifluoromethyl group, a pentafluoroethyl group, a heptafluoropropyl group, a trichloromethyl group, a tribromomethyl group, a triiodomethyl group, and the like.

The phenyl group, the naphthyl group or the benzyl group, having a substituent, in $R_1$ to $R_4$ of the general formula (1), has one to five substituents, and preferably one to three substituents. The substituent includes, for example, an alkyl group having 1 to 6 carbon atoms such as a methyl group, an ethyl group, a propyl group, an isopropyl group, a butyl group, an isobutyl group, a tert-butyl group, a pentyl group and a hexyl group; a halogeno group such as a fluoro group, a chloro group, a bromo group and an iodo group; an alkoxy group having 1 to 6 carbon atoms such as a methoxy group, an ethoxy group, a propoxy group, a butoxy group, a tert-butoxy group, a propoxy group and a hexyloxy group; a hydroxyalkyl group having 1 to 6 carbon atoms such as a hydroxyethyl group and a hydroxypropyl group; an alkoxyalkyl group having 2 to 7 carbon atoms such as a methoxyethyl group, an ethoxyethyl group, an ethoxypropyl group and a butoxyethyl group; a hydroxyalkoxy group having 1 to 6 carbon atoms such as a 2-hydroxyethoxy group; an alkoxyalkoxy group having 2 to 7 carbon atoms such as a 2-methoxyethoxy group and a 2-ethoxyethoxy group; a sulfoalkyl group having 1 to 6 carbon atoms such as 2-sulfoethyl group; a carboxyalkyl group having 2 to 7 carbon atoms such as a carboxymethyl group, a carboxyethyl group, a carboxypropyl group, a carboxybutyl group, a carboxypentyl group and a carboxyhexyl group; a cyanoalkyl group having 2 to 7 carbon atoms such as a cyanomethyl group, a cyanoethyl group, a cyanopropyl group, a cyanobutyl group, a cyanopentyl group and a cyanohexyl group; a sulfo group; and the like.

As $R_1$ to $R_4$ in the general formula (1), an alkyl group having 1 to 30 carbon atoms is preferable, and an alkyl group having 1 to 6 carbon atoms is more preferable. Specifically, a methyl group, an ethyl group, a propyl group, an isopropyl group, a butyl group, a pentyl group and a hexyl group are preferable, and a methyl group, an ethyl group and a propyl group are more preferable, and a methyl group and an ethyl group are particularly preferable.

$R_5$ and $R_6$ in the general formula (1) represent a hydrogen atom or a methyl group, and a hydrogen atom is preferable.

$R_7$ in the general formula (1) represents a hydrogen atom or a methyl group, and a methyl group is preferable.

The halogen atom in $R_8$ of the general formula (1) includes a fluorine atom, a chlorine atom, a bromine atom, an iodine atom, and the like, and among them, a bromine atom is preferable.

The alkyl group having 1 to 21 carbon atoms, in $R_8$ of the general formula (1), may be any of the linear, branched, or cyclic one, and the one having 1 to 12 carbon atoms is preferable, and the one having 1 to 6 carbon atoms is more preferable, and the one having 1 to 3 carbon atoms is further preferable. Specifically it includes, for example, a methyl group, an ethyl group, a propyl group, an isopropyl group, a butyl group, a 1-methylpropyl group, an isobutyl group, a tert-butyl group, a pentyl group, a 1-ethylpropyl group, a 1-methylbutyl group, a cyclopentyl group, a hexyl group, a 1-methylpentyl group, a 1-ethylbutyl group, a cyclohexyl group, a 2-heptyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a henicosyl group, an isopentyl group, an isohexyl group, an isoheptyl group, an isooctyl group, an isononyl group, an isodecyl group, an isoundecyl group, an isododecyl group, an isotridecyl group, an isotetradecyl group, an isopentadecyl group, an isohexadecyl group, an isoheptadecyl group, an isooctadecyl group, an isononadecyl group, an isoicosyl group, an isohenicosyl group, a 1-methylhexyl group, a 2-ethylhexyl group, a 1-methylheptyl group, a 1-cyclohexylethyl group, a 1-heptyloctyl group, a 1-ethylheptyl group, a 2-methylcyclohexyl group, a 3-methylcyclohexyl group, a 4-methylcyclohexyl group, a 2,6-dimethylcyclohexyl group, a 2,4-dimethylcyclohexyl group, a 3,5-dimethylcyclohexyl group, a 2,5-dimethylcyclohexyl group, a 2,3-dimethylcyclohexyl group, a 3,3,5-trimethylcyclohexyl group, a 4-tert-butylcyclohexyl group, a 2-ethylhexyl group, and the like; and a methyl group, an ethyl group, a propyl group, an isopropyl group, a butyl group, a pentyl group and a hexyl group are preferable; and a methyl group, an ethyl group and a propyl group are more preferable.

As the aryl group having 6 to 10 carbon atoms in $R_8$ of the general formula (1), a phenyl group, a naphthyl group, and the like, is included, and a phenyl group is preferable.

As the alkoxy group having 1 to 3 carbon atoms in $R_8$ of the general formula (1), a methoxy group, an ethoxy group, a n-propoxy group, an iso-propoxy group, and the like, is included.

As $R_8$ in the general formula (1), a halogen atom, a hydroxy group, a nitro group and an alkoxy group having 1 to 3 carbon atoms are preferable.

As n in the general formula (1), 0 to 1 is preferable, and 0 is more preferable.

As the arylene group in "an alkylene group having 1 to 21 carbon atoms, which has at least one group selected from -O-, -OCO-, -COO- and an arylene group in the chain" and "an alkylene group having 1 to 21 carbon atoms, which has at least one group selected from -O-, -OCO-, -COO- and an arylene group in the chain, and also has a hydroxy group as a substituent", in $A_1$ of the general formula (1), a phenylene group and a naphthylene group are included, and a phenylene group is preferable.

As the alkylene group having 1 to 21 carbon atoms in "an alkylene group having 1 to 21 carbon atoms, which has at least one group selected from -O-, -OCO-, -COO- and an arylene group in the chain", "an alkylene group having 1 to 21 carbon atoms, which has at least one group selected from -O-, -OCO-, -COO-and an arylene group in the chain, and also has a hydroxy group as a substituent", "an alkylene group having 1 to 21 carbon atoms which has a hydroxy group as a substituent" and "an alkylene group having 1 to 21 carbon atoms", in $A_1$ of the general formula (1), the linear or branched one is preferable; and the one having 1 to 12 carbon atoms is preferable, and the one having 1 to 6 carbon atoms is more preferable, and the one having 1 to 3 carbon atoms is further preferable. Specifically it includes, for example, a methylene group, an ethylene group, a propylene group, a methylethylene group, a butylene group, a 1-methylpropylene group, a 2-methylpropylene group, a pentylene group, a methylbutylene group, a 1,2-dimethylpropylene group, a 1-ethylpropylene group, a hexylene group, a methylpentylene group, an n-heptylene group, an n-octylene group, an n-nonylene group, an n-decylene group, an n-undecylene group, an n-dodecylene group, an n-tridecylene group, an n-tetradecylene group, an n-pentadecylene group, an n-hexadecylene group, an n-heptadecylene group, an n-octadecylene group, an n-nonadecylene group, an n-icosylene group, an n-henicosylene group, and the like, and a methylene group, an ethylene group, a propylene group, a butylene group, a pentylene group and a hexylene group are preferable, and a methylene group, an ethylene group and a propylene group are more preferable, and an ethylene group is particularly preferable.

"An alkylene group having 1 to 21 carbon atoms, which has at least one group selected from -O-, -OCO-, -COO- and an arylene group in the chain", in $A_1$ of the general formula (1), includes, for example, the groups represented by the following general formulae (21-1) and (21-2), and the like.

$$-(R_{51}\text{-}O)_{h1}\text{-}R_{52}\text{-} \qquad (21\text{-}1)$$

(wherein $R_{51}$ and $R_{52}$ each independently represent a linear or branched alkylene group having 1 to 4 carbon atoms; and $h_1$ represents an integer of 1 to 9; provided that total number of carbon atoms in the formula is 1 to 21.)

$$-(CH_2)_{h2}\text{-OCO-}R_{53}\text{-COO-}(CH_2)_{h3}\text{-} \qquad (21\text{-}2)$$

(wherein $R_{53}$ represents a phenylene group or an alkylene group having 1 to 7 carbon atoms; $h_2$ and $h_3$ each independently represent an integer of 1 to 3.)

The linear or branched alkylene group having 1 to 4 carbon atoms, in $R_{51}$ and $R_{52}$ of the general formula (21-1), specifically includes, for example, a methylene group, an ethylene group, a methylmethylene group, a propylene group, a methylethylene group, a butylene group, a methylpropylene group, and the like, and an ethylene group and a methylethylene group are preferable.

The group represented by the general formula (21-1), specifically includes, for example,

$-CH_2CH_2\text{-O-}CH_2CH_2\text{-}$,
$-(CH_2CH_2\text{-O})_2\text{-}CH_2CH_2\text{-}$,
$-(CH_2CH_2\text{-O})_3\text{-}CH_2CH_2\text{-}$,
$-(CH_2CH_2\text{-O})_4\text{-}CH_2CH_2\text{-}$,

$-(CH_2CH_2-O)_5-CH_2CH_2-$,
$-(CH_2CH_2-O)_6-CH_2CH_2-$,
$-(CH_2CH_2-O)_7-CH_2CH_2-$,
$-(CH_2CH_2-O)_8-CH_2CH_2-$,
$-(CH_2CH_2-O)_9-CH_2CH_2-$,
$-CH_2CH(CH_3)-O-CH_2CH(CH_3)-$,
$-(CH_2CH(CH_3)-O)_2-CH_2CH(CH_3)-$,
$-(CH_2CH(CH_3)-O)_3-CH_2CH(CH_3)-$,
$-(CH_2CH(CH_3)-O)_4-CH_2CH(CH_3)-$,
$-(CH_2CH(CH_3)-O)_5-CH_2CH(CH_3)-$,
$-(CH_2CH(CH_3)-O)_6-CH_2CH(CH_3)-$,
$-CH(CH_3)CH_2-O-CH(CH_3)CH_2-$,
$-(CH(CH_3)CH_2-O)_2-CH(CH_3)CH_2-$,
$-(CH(CH_3)CH_2-O)_3-CH(CH_3)CH_2-$,
$-(CH(CH_3)CH_2-O)_4-CH(CH_3)CH_2-$,
$-(CH(CH_3)CH_2-O)_5-CH(CH_3)CH_2-$,
$-(CH(CH_3)CH_2-O)_6-CH(CH_3)CH_2-$,
$-CH(CH_3)CH_2-O-CH_2CH(CH_3)-$,

and the like.

The alkylene group having 1 to 7 carbon atoms, in $R_{53}$ of the general formula (21-2), specifically includes, for example, a methylene group, an ethylene group, a propylene group, a butylene group, a pentylene group, a hexylene group, a heptylene group, a cyclobutylene group, a cyclopentylene group, a cyclohexylene group, a cycloheptylene group, and the like.

The group represented by the general formula (21-2), specifically includes, for example,

$-CH_2-O-CO-CH_2-CO-O-CH_2-$,
$-CH_2-O-CO-(CH_2)_2-CO-O-CH_2-$,
$-CH_2-O-CO-(CH_2)_3-CO-O-CH_2-$,
$-CH_2-O-CO-(CH_2)_4-CO-O-CH_2-$,
$-CH_2-O-CO-(CH_2)_5-CO-O-CH_2-$,
$-CH_2-O-CO-(CH_2)_6-CO-O-CH_2-$,
$-CH_2-O-CO-(CH_2)_7-CO-O-CH_2-$,
$-(CH_2)_2-O-CO-CH_2-CO-O-(CH_2)_2-$,
$-(CH_2)_2-O-CO-(CH_2)_2-CO-O-(CH_2)_2-$,
$-(CH_2)_2-O-CO-(CH_2)_3-CO-O-(CH_2)_2-$,
$-(CH_2)_2-O-CO-(CH_2)_4-CO-O-(CH_2)_2-$,
$-(CH_2)_2-O-CO-(CH_2)_5-CO-O-(CH_2)_2-$,
$-(CH_2)_2-O-CO-(CH_2)_6-CO-O-(CH_2)_2-$,
$-(CH_2)_2-O-CO-(CH_2)_7-CO-O-(CH_2)_2-$,
$-(CH_2)_3-O-CO-CH_2-CO-O-(CH_2)_3-$,
$-(CH_2)_3-O-CO-(CH_2)_2-CO-O-(CH_2)_3-$,
$-(CH_2)_3-O-CO-(CH_2)_3-CO-O-(CH_2)_3-$,
$-(CH_2)_3-O-CO-(CH_2)_4-CO-O-(CH_2)_3-$,
$-(CH_2)_3-O-CO-(CH_2)_5-CO-O-(CH_2)_3-$,
$-(CH_2)_3-O-CO-(CH_2)_6-CO-O-(CH_2)_3-$,
$-(CH_2)_3-O-CO-(CH_2)_7-CO-O-(CH_2)_3-$,
$-CH_2-O-CO-C_6H_4-CO-O-CH_2-$,
$-(CH_2)_2-O-CO-C_6H_4-CO-O-(CH_2)_2-$,
$-(CH_2)_3-O-CO-C_6H_4-CO-O-(CH_2)_3-$,
$-CH_2-O-CO-C_6H_{10}-CO-O-CH_2-$,
$-(CH_2)_2-O-CO-C_6H_{10}-CO-O-(CH_2)_2-$,
$-(CH_2)_3-O-CO-C_6H_{10}-CO-O-(CH_2)_3-$,

and the like. Among them

$-CH_2-O-CO-CH_2-CO-O-CH_2-$,
$-CH_2-O-CO-(CH_2)_2-CO-O-CH_2-$,

$-CH_2-O-CO-(CH_2)_3-CO-O-CH_2-$,
$-CH_2-O-CO-(CH_2)_4-CO-O-CH_2-$,
$-CH_2-O-CO-(CH_2)_5-CO-O-CH_2-$,
$-CH_2-O-CO-(CH_2)_6-CO-O-CH_2-$,
$-CH_2-O-CO-(CH_2)_7-CO-O-CH_2-$,
$-(CH_2)_2-O-CO-CH_2-CO-O-(CH_2)_2-$,
$-(CH_2)_2-O-CO-(CH_2)_2-CO-O-(CH_2)_2-$,
$-(CH_2)_2-O-CO-(CH_2)_3-CO-O-(CH_2)_2-$,
$-(CH_2)_2-O-CO-(CH_2)_4-CO-O-(CH_2)_2-$,
$-(CH_2)_2-O-CO-(CH_2)_5-CO-O-(CH_2)_2-$,
$-(CH_2)_2-O-CO-(CH_2)_6-CO-O-(CH_2)_2-$,
$-(CH_2)_2-O-CO-(CH_2)_7-CO-O-(CH_2)_2-$,
$-(CH_2)_3-O-CO-CH_2-CO-O-(CH_2)_3-$,
$-(CH_2)_3-O-CO-(CH_2)_2-CO-O-(CH_2)_3-$,
$-(CH_2)_3-O-CO-(CH_2)_3-CO-O-(CH_2)_3-$,
$-(CH_2)_3-O-CO-(CH_2)_4-CO-O-(CH_2)_3-$,
$-(CH_2)_3-O-CO-(CH_2)_5-CO-O-(CH_2)_3-$,
$-(CH_2)_3-O-CO-(CH_2)_6-CO-O-(CH_2)_3-$ and
$-(CH_2)_3-O-CO-(CH_2)_7-CO-O-(CH_2)_3-$

are preferable, and

$-(CH_2)_2-O-CO-CH_2-CO-O-(CH_2)_2-$,
$-(CH_2)_2-O-CO-(CH_2)_2-CO-O-(CH_2)_2-$,
$-(CH_2)_2-O-CO-(CH_2)_3-CO-O-(CH_2)_2-$,
$-(CH_2)_2-O-CO-(CH_2)_4-CO-O-(CH_2)_2-$,
$-(CH_2)_2-O-CO-(CH_2)_5-CO-O-(CH_2)_2-$,
$-(CH_2)_2-O-CO-(CH_2)_6-CO-O-(CH_2)_2-$ and
$-(CH_2)_2-O-CO-(CH_2)_7-CO-O-(CH_2)_2-$

are more preferable, and

$-(CH_2)_2-O-CO-(CH_2)_2-CO-O-(CH_2)_2-$

is particularly preferable.

"An alkylene group having 1 to 21 carbon atoms, which has at least one group selected from -O-, -OCO-, -COO- and an arylene group in the chain, and also has a hydroxy group as a substituent", in $A_1$ of the general formula (1), includes, for example, the groups represented by the following general formulae (22-1) and (22-2).

$$-R_{54}-(CH_2)_{h4}- \quad (22\text{-}1)$$

(wherein $R_{54}$ represents an arylene group having 6 to 10 carbon atoms which has a hydroxy group as a substituent; and $h_4$ represents an integer of 1 to 4.)

$$-R_{55}-Y_1-(CH_2)_{h5}- \quad (22\text{-}2)$$

(wherein $R_{55}$ represents an alkylene group having 1 to 7 carbon atoms which has a hydroxy group as a substituent, or an arylene group having 6 to 10 carbon atoms which has a hydroxy group as a substituent; $Y_1$ represents -O-, -OCO- or -COO-; and $h_5$ represents an integer of 2 to 4.)

The arylene group having 6 to 10 carbon atoms which has a hydroxy group as a substituent, in $R_{54}$ of the general formula (22-1), includes a hydroxyphenylene group, a dihydroxyphenylene group, a hydroxynaphthylene group, a dihydroxynaphthylene group, and the like.

Preferable specific examples of the group represented by the general formula (22-1) include, for example,

$-C_6H_3(OH)-CH_2-$, $-C_6H_3(OH)-(CH_2)_2-$,
$-C_sH_3(OH)-(CH_2)_3-$, $-C_6H_3(OH)-(CH_2)_4-$,
$-C_6H_2(OH)_2-CH_2-$, $-C_6H_2(OH)_2-(CH_2)_2-$,
$-C_6H_2(OH)_2-(CH_2)_3-$, $-C_6H_2(OH)_2-(CH_2)_4-$,

and the like.

The alkylene group having 1 to 7 carbon atoms which has a hydroxy group as a substituent, in $R_{55}$ of the general formula (22-2), includes a hydroxymethylene group, a hydroxyethylene group, a hydroxypropylene group, a hydroxybutylene group, a hydroxypentylene group, a hydroxyhexylene group, a hydroxycyclobutylene group, a hydroxycyclopentylene group, a hydroxycyclohexylene group, a hydroxycycloheptylene group, and the like.

The arylene group having 6 to 10 carbon atoms which has a hydroxy group as a substituent, in $R_{55}$ of the general formula (22-2), includes the same one as the arylene group having 6 to 10 carbon atoms which has a hydroxy group as a substituent, in $R_{54}$ of the general formula (22-1).

Preferable specific examples of the group represented by the general formula (22-2) include, for example,

$-CH_2-CH(OH)-CH_2-O-(CH_2)_2-$,
$-CH_2-CH(OH)-CH_2-O-(CH_2)_3-$,
$-CH_2-CH(OH)-CH_2-O-(CH_2)_4-$,
$-CH_2-CH(OH)-CH_2-OCO-(CH_2)_2-$,
$-CH_2-CH(OH)-CH_2-OCO-(CH_2)_3-$,
$-CH_2-CH(OH)-CH_2-OCO-(CH_2)_4-$,
$-CH_2-CH(OH)-CH_2-COO-(CH_2)_2-$,
$-CH_2-CH(OH)-CH_2-COO-(CH_2)_3-$,
$-CH_2-CH(OH)-CH_2-COO-(CH_2)_4-$,

and the like.

"An alkylene group having 1 to 21 carbon atoms which has a hydroxy group as a substituent" in $A_1$ of the general formula (1) includes, for example, the group represented by the following general formula (23-1), and the like.

$$-R_{56}-(CH_2)_{h6}- \qquad (23\text{-}1)$$

(wherein $R_{56}$ represents an alkylene group having 1 to 7 carbon atoms which has a hydroxy group as a substituent; and $h_6$ represents an integer of 1 to 4.)

The alkylene group having 1 to 7 carbon atoms which has a hydroxy group as a substituent, in $R_{56}$ of the general formula (23-1), includes the same one as the alkylene groups having 1 to 7 carbon atoms which has a hydroxy group as a substituent, in $R_{55}$ of the general formula (22-2).

The group represented by the general formula (23-1) specifically include, for example,

$-CH_2-CH(OH)-CH_2-$,
$-CH_2-CH(OH)-(CH_2)_2-$,
$-CH_2-CH(OH)-(CH_2)_3-$,
$-CH_2-CH(OH)-(CH_2)_4-$,
$-C_6H_9(OH)-CH_2-$,
$-C_6H_9(OH)-(CH_2)_2-$,
$-C_6H_9(OH)-(CH_2)_3-$,
$-C_6H_9(OH)-(CH_2)_4-$,

and the like.

As $A_1$ in the general formula (1), an alkylene group having 1 to 21 carbon atoms is preferable. Among them, a methylene group, an ethylene group, a propylene group, a butylene group, a pentylene group and a hexylene group are preferable, and a methylene group, an ethylene group and a propylene group are more preferable, and an ethylene group is particularly preferable.

As $A_2$ in the general formula (1), -O- is preferable.

The group represented by the following general formula (1-1), bonding to a phenyl group in a fundamental skeleton of the triphenylmethane derivative, in the general formula (1), may be located at any of ortho position, meta position or para position of the phenyl group, and para position is preferable. Specifically, the one is preferable where the group represented by the general formula (1-1) bonds to the phenyl group in the fundamental skeleton of the triphenylmethane derivative, as the compound represented by the following general formula (1-2).

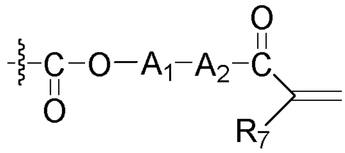

(1-1)

(wherein $R_7$, $A_1$ and $A_2$ are the same as described above.)

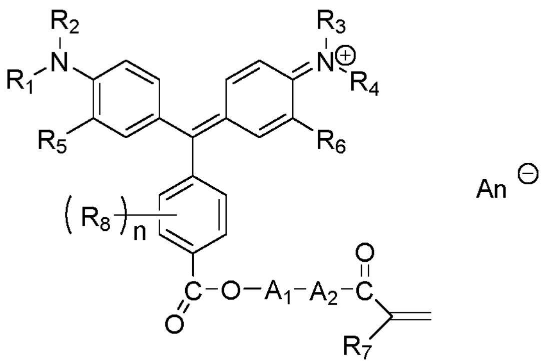

(1-2)

(wherein $R_1$ to $R_8$, n, $A_1$, $A_2$ and $An^-$ are the same as described above.)

Preferable specific examples of the compound of the present invention include the compound represented by the following general formula (1').

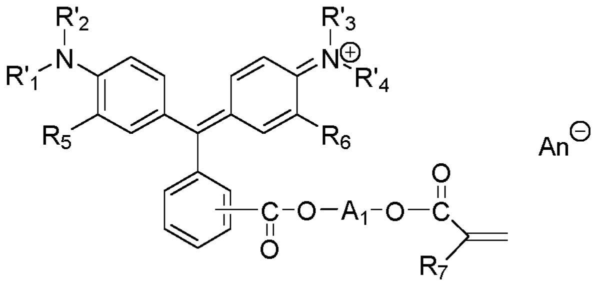

(1')

(wherein $R'_1$ to $R'_4$ each independently represent an alkyl group having 1 to 30 carbon atoms; and $R_5$ to $R_7$, $A_1$ and $An^-$ are the same as described above.)

The alkyl group having 1 to 30 carbon atoms, in $R'_1$ to $R'_4$ of the general formula (1'), includes the same one as the alkyl group having 1 to 30 carbon atoms, in $R_1$ to $R_4$ of the general formula (1), and the preferable ones are also the same.

As $R'_1$ to $R'_4$ in the general formula (1'), an alkyl group having 1 to 6 carbon atoms is preferable. Specifically, a methyl group, an ethyl group, a propyl group, an isopropyl group, a butyl group, a pentyl group and a hexyl group are preferable, and a methyl group, an ethyl group and a propyl group are more preferable, and a methyl group and an ethyl group are particularly preferable.

Preferable specific examples among the compound represented by the general formula (1') include the compound represented by the following general formula (1").

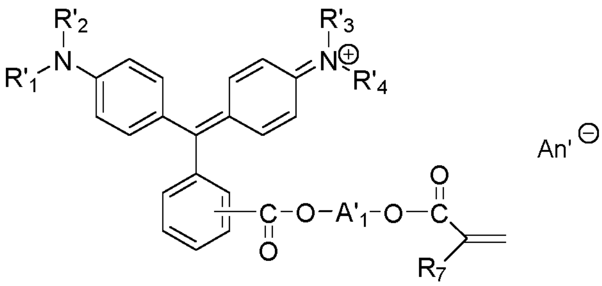

(1")

(wherein $A'_1$ represents an alkylene group having 1 to 21 carbon atoms; $An'^-$ represents an anion containing an aryl group having a halogeno group, a sulfonyl group having a halogeno group, or a halogenated alkyl group; and $R'_1$ to $R'_4$ and $R_7$ are the same as described above.)

The alkylene group having 1 to 21 carbon atoms in $A'_1$ of the general formula (1") includes the same one as the alkylene group having 1 to 21 carbon atoms in $A_1$ of the general formula (1), and the preferable ones are also the same.

An anion moiety in the anion containing the aryl group having a halogeno group, the sulfonyl group having a halogeno group or the halogenated alkyl group, represented by $An'^-$ of the general formula (1"), includes the same one as the anion moiety in the anion of the present invention, and the preferable ones are also the same.

The halogeno group in the aryl group having a halogeno group, or the sulfonyl group having a halogeno group, represented by $An'^-$ of the general formula (1"), includes, for example, a fluoro group, a chloro group, a bromo group or an iodo group, and a fluoro group is preferable.

The aryl group in the aryl group having a halogeno group, represented by $An'^-$ of the general formula (1"), includes, for example, a phenyl group, a naphthyl group, and the like, and a phenyl group is preferable.

Specific examples of the aryl group having a halogeno group, represented by $An'^-$ of the general formula (1"), include, for example, a monofluorophenyl group, a difluorophenyl group, a trifluorophenyl group, a perfluorophenyl group, a monochlorophenyl group, a dichlorophenyl group, a trichlorophenyl group, a perchlorophenyl group, a monobromophenyl group, a dibromophenyl group, a tribromophenyl group, a perbromophenyl group, a monoiodophenyl group, a diiodophenyl group, a triiodophenyl group, a periodophenyl group, a monofluoronaphthyl group, a difluoronaphthyl group, a trifluoronaphthyl group, a perfluoronaphthyl group, a monochloronaphthyl group, a dichloronaphthyl group, a trichloronaphthyl group, a perchloronaphthyl group, a monobromonaphthyl group, a dibromonaphthyl group, a tribromonaphthyl group, a perbromonaphthyl group, a monoiodonaphthyl group, a diiodonaphthyl group, a triiodonaphthyl group, a periodonaphthyl group, and the like; and a monofluorophenyl group, a difluorophenyl group, a trifluorophenyl group, a perfluorophenyl group, a monochlorophenyl group, a dichlorophenyl group, a trichlorophenyl group, a perchlorophenyl group, a monobromophenyl group, a dibromophenyl group, a tribromophenyl group, a perbromophenyl group, a monoiodophenyl group, a diiodophenyl group, a triiodophenyl group and a periodophenyl group are preferable; and a difluorophenyl group, a trifluorophenyl group and a perfluorophenyl group are more preferable; and a perfluorophenyl group is particularly preferable.

The sulfonyl group having a halogeno group, represented by $An'^-$ of the general formula (1"), includes, for example, $-SO_2-F$, $-SO_2-Cl$, $-SO_2-Br$, $-SO_2-I$, and the like.

The halogenated alkyl group represented by $An'^-$ of the general formula (1") includes the same one as the halogenated alkyl group in the anion of the present invention, and the preferable ones are also the same.

The anions containing the aryl group having a halogeno group, the sulfonyl group having a halogeno group, or the halogenated alkyl, represented by $An'^-$ of the general formulae (1"), specifically include, for example, those represented by the following general formulae (13') to (18').

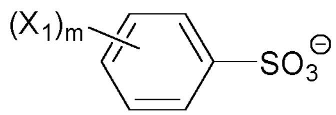

(13')

(wherein m is the same as described above; and m pieces of $X_1$ each independently represent a halogen atom.)

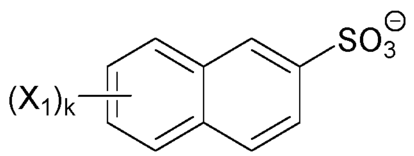

(14')

(wherein $X_1$ and k are the same as described above; and k pieces of $X_1$ may be the same or different.)

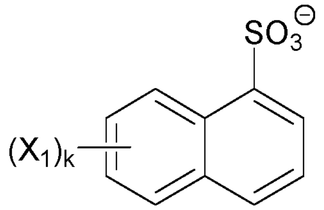

(15')

(wherein $X_1$ and k are the same as described above; and k pieces of $X_1$ may be the same or different.)

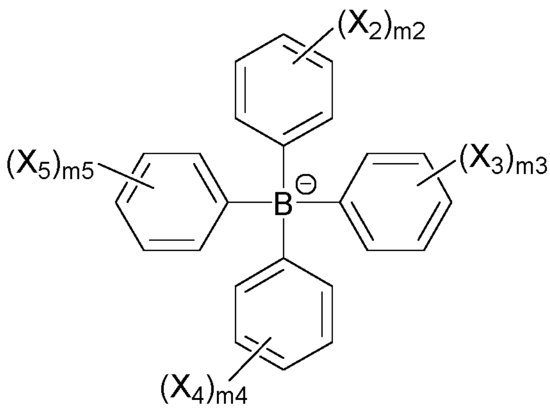

(16')

(wherein $X_2$ to $X_5$ each independently represent a halogen atom; $m_2$ to $m_5$ are the same as described above; and $m_2$ pieces of $X_2$, $m_3$ pieces of $X_3$, $m_4$ pieces of $X_4$ and $m_5$ pieces of $X_5$ may each be the same or different.)

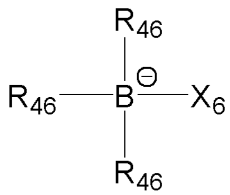

(17')

(wherein $X_6$ represents a halogen atom; and $R_{46}$ is the same as described above; provided that at least one of three $R_{46}$ represents a halogenated alkyl group having 1 to 3 carbon atoms.)

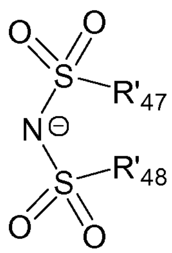

(18')

(wherein $R'_{47}$ and $R'_{48}$ each independently represent a halogen atom, or $R'_{47}$ together with $R'_{48}$ form a halogenated alkylene group having 2 to 3 carbon atoms.)

The halogen atom in $X_1$ to $X_6$, $R'_{47}$ and $R'_{48}$ of the general formulae (13') to (18'), includes a fluorine atom, a chlorine atom, a bromine atom, an iodine atom, and the like, and among them, a fluorine atom is preferable.

The m pieces of the $X_1$ in the general formula (13') are each independent, and it is preferable that they are all the same.

Preferable specific examples of the anion represented by the general formula (13') include, for example, the following ones.

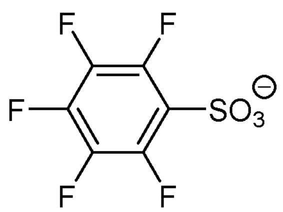

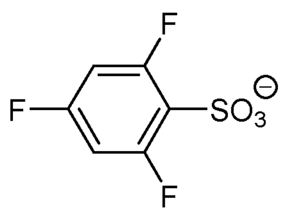

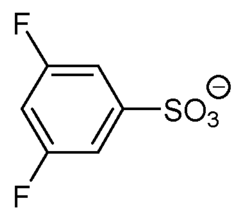

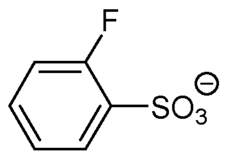
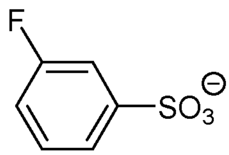
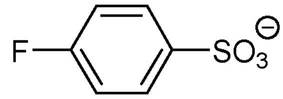

The k pieces of the $X_1$ in the general formulae (14') and (15') are each independent, and it is preferable that they are all the same.

Preferable specific examples of the anion represented by the general formulae (14') and (15') include, for example, the following ones.

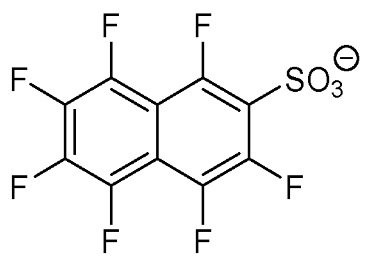
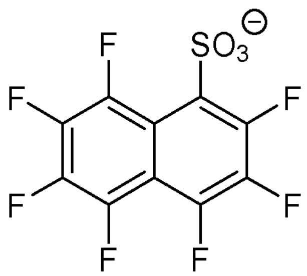

The $m_2$ pieces of $X_2$, $m_3$ pieces of $X_3$, $m_4$ pieces of $X_4$ and $m_5$ pieces of $X_5$ in the general formula (16') are each independent, and it is preferable that they are all the same.

Preferable specific examples of the anion represented by the general formula (16') include, for example, the following ones.

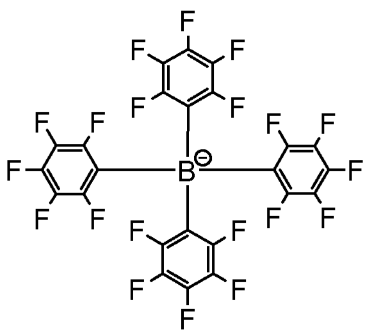
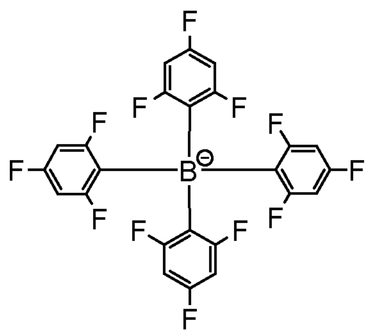
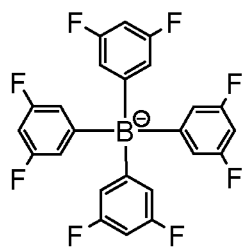

Among the specific examples, the following one is more preferable.

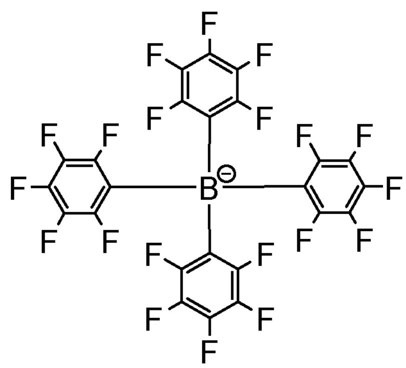

Preferable specific examples of the anion represented by a general formula (17') include, for example, $CF_3BF_3^-$, $C_2F_5BF_3^-$, $C_3F_7BF_3^-$, and the like.

The halogenated alkylene group having 2 to 3 carbon atoms, formed by $R'_{47}$ together with $R'_{48}$ of the general formula (18'), includes, for example, a tetrafluoroethylene group, a hexafluoropropylene group and the like, and a hexafluoropropylene group is preferable.

Preferable specific examples of the anion represented by the general formula (18') include, for example, the following ones.

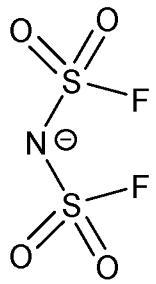
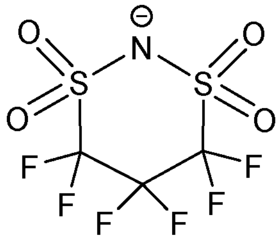

As the anion containing the aryl group having a halogeno group, the sulfonyl group having a halogeno group or the halogenated alkyl group, represented by An'$^-$ of the general formula (1"), the one represented by the general formula (16'), the general formula (17'), or the general formula (18') is preferable, and the one represented by the general formula (16'), or the general formula (18') is more preferable, and the one represented by the general formula (16') is particularly preferable.

Among the specific examples of the anion containing the aryl group having a halogeno group, the sulfonyl group having a halogeno group or the halogenated alkyl group, represented by An'$^-$ of the general formula (1"), the following ones are more preferable.

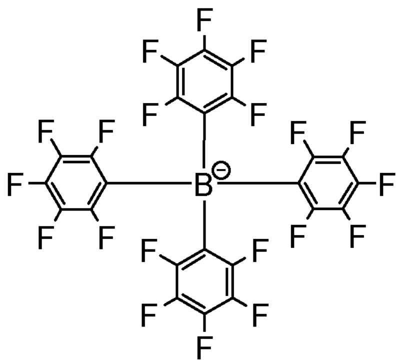
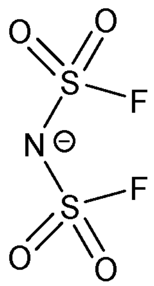
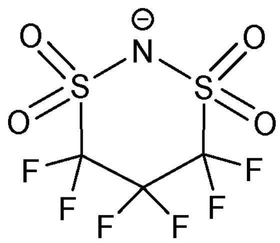

Among the specific examples, the following one is more preferable.

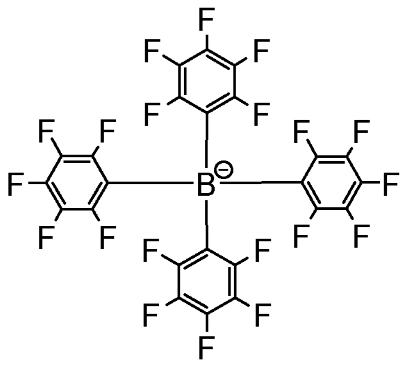

Preferable combinations of $R'_1$ to $R'_4$, $R_7$ and $A'_1$ in the general formula (1") include, for example, those described in the following table.

| $R_1$' | $R_2$' | $R_3$' | $R_4$' | $R_7$ | $A_1$' |
|---|---|---|---|---|---|
| Hydrogen atom | Hydrogen atom | Hydrogen atom | Hydrogen atom | Hydrogen atom or methyl group | Ethylene group |

(continued)

| $R_1'$ | $R_2'$ | $R_3'$ | $R_4'$ | $R_7$ | $A_1'$ |
|---|---|---|---|---|---|
| Hydrogen atom | Methyl group | Methyl group | Hydrogen atom | Hydrogen atom or methyl group | Ethylene group |
| Hydrogen atom | Ethyl group | Ethyl group | Hydrogen atom | Hydrogen atom or methyl group | Ethylene group |
| Hydrogen atom | n-propyl group | n-propyl group | Hydrogen atom | Hydrogen atom or methyl group | Ethylene group |
| Hydrogen atom | Isopropyl group | Isopropyl group | Hydrogen atom | Hydrogen atom or methyl group | Ethylene group |
| Methyl group | Methyl group | Methyl group | Methyl group | Hydrogen atom or methyl group | Ethylene group |
| Methyl group | Ethyl group | Ethyl group | Methyl group | Hydrogen atom or methyl group | Ethylene group |
| Methyl group | n-propyl group | n-propyl group | Methyl group | Hydrogen atom or methyl group | Ethylene group |
| Methyl group | Isopropyl group | Isopropyl group | Methyl group | Hydrogen atom or methyl group | Ethylene group |
| Ethyl group | Ethyl group | Ethyl group | Ethyl group | Hydrogen atom or methyl group | Methylene group |
| Ethyl group | Ethyl group | Ethyl group | Ethyl group | Hydrogen atom or methyl group | Ethylene group |
| Ethyl group | Ethyl group | Ethyl group | Ethyl group | Hydrogen atom or methyl group | Propylene group |
| Ethyl group | n-propyl group | n-propyl group | Ethyl group | Hydrogen atom or methyl group | Ethylene group |
| Ethyl group | Isopropyl group | Isopropyl group | Ethyl group | Hydrogen atom or methyl group | Ethylene group |
| n-propyl group | n-propyl group | n-propyl group | n-propyl group | Hydrogen atom or methyl group | Ethylene group |

$An'^{-}$ used together with the combinations in the table includes, for example, the following ones.

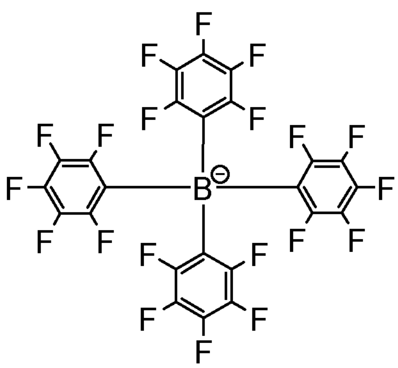

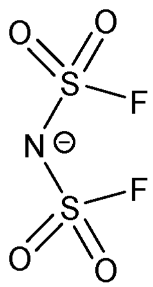

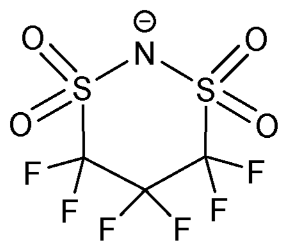

<u>[Production method for the compound of the present invention]</u>

The compound of the present invention is produced by sequentially carrying out the following reactions [I] to [IV].

[I] A compound represented by the following general formula (31) and a compound represented by the following general formula (32) are reacted.

[II] A compound obtained in the reaction [I] is further reacted with a compound represented by the following general formula (33) to obtain a compound represented by the following general formula (34).

[III] The compound represented by the general formula (34) and a compound represented by the following general formula (35) are reacted in the presence of a dehydration condensation agent to obtain a triphenylmethane-based compound represented by the following general formula (36).

[IV] The triphenylmethane-based compound represented by the general formula (36) is subjected to an oxidation reaction and a salt exchange reaction to obtain the compound of the present invention represented by the general formula (1).

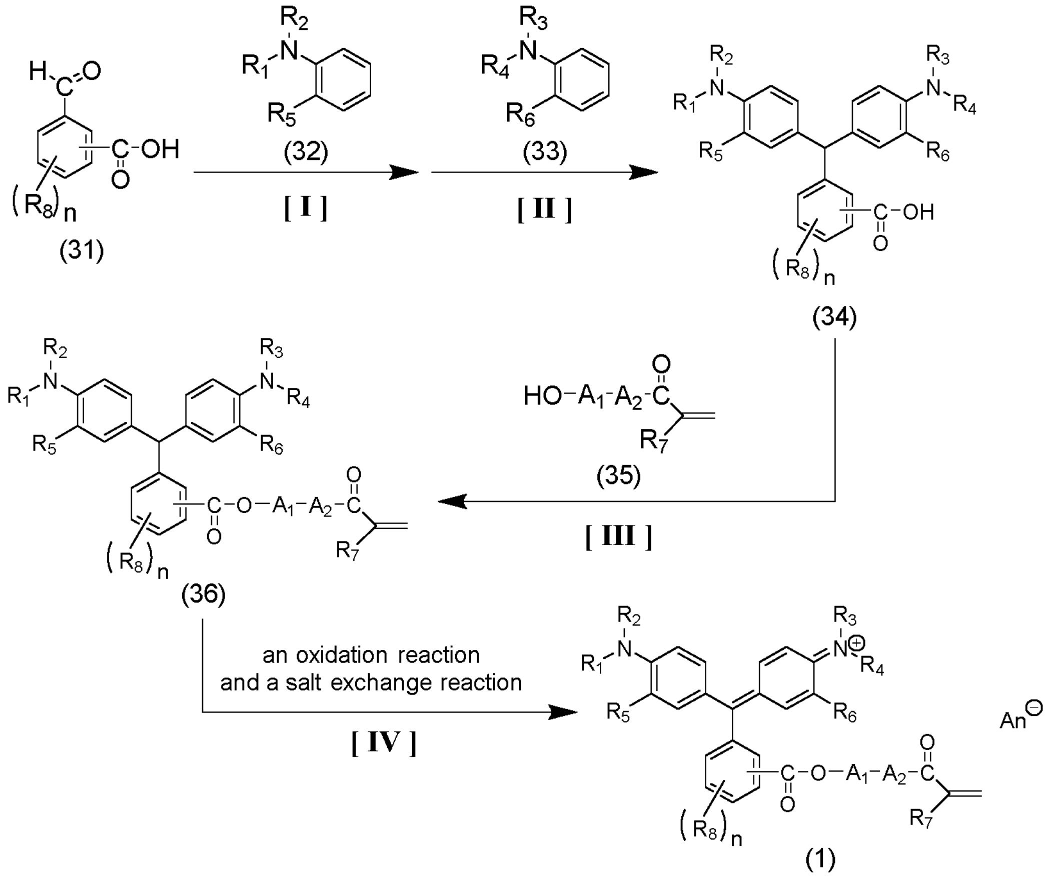


(wherein $R_1$ to $R_8$, n, $A_1$, $A_2$ and $An^-$ are the same as described above.)

In the reaction [I], the compound represented by the general formula (31), and the compound represented by the general formula (32) may be reacted in a solvent, in the presence of an acid catalyst, usually at 80 to 150°C, preferably at 100 to 130°C, for usually 1 to 24 hours, and preferably 5 to 15 hours.

The acid catalyst includes sulfuric acid, methane sulfonic acid, trifluoromethane sulfonic acid, p-toluene sulfonic acid, camphor sulfonic acid, and the like, and p-toluene sulfonic acid is preferable. Use amount of the acid catalyst is usually 0.1 to 10 equivalents, and preferably 0.5 to 2 equivalents, relative to mole number of the compound represented by the general formula (31).

The solvent includes an organic solvent such as ketones, for example, diethyl ketone, methyl isobutyl ketone (MIBK), and the like; and ethers, for example, diisopropyl ether, and the like. Among them, MIBK is preferable. They may be used singly, or in combination of two or more kinds thereof as appropriate. Use amount of the reaction solvent is usually 1 to 20 times, and preferably 1 to 5 times, relative to total weight of the compound represented by the general formula (31) and the compound represented by the general formula (32).

Use amount of the compound represented by the general formula (32) is usually 1 to 3 equivalents, and preferably 1 to 2 equivalents, relative to mole number of the compound represented by the general formula (31).

Specific examples of the compound represented by the general formulae (31) include the following ones.

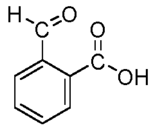 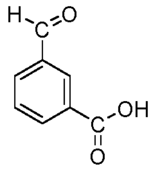 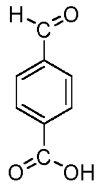 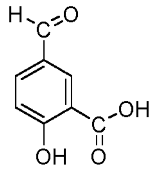 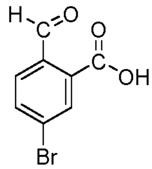 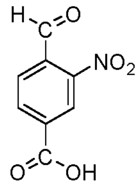 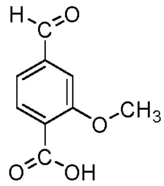

The compound represented by the general formula (32) includes, for example, N-methylaniline, N-ethylaniline, N-propylaniline, N,N-dimethylaniline, N,N-diethylaniline, N,N-dipropylaniline, N,N-dimethyl-o-toluidine, N,N-diethyl-o-toluidine, N,N-dipropyl-o-toluidine, and the like, and N-methylaniline, N-ethylaniline, N-propylaniline, N,N-dimethylaniline, N,N-diethylaniline and N,N-dipropylaniline are preferable, and N,N-diethylaniline is more preferable.

In the reaction [II], the compound obtained in the reaction [I] and the compound represented by the general formula (33) may be reacted under reaction conditions (a reaction solvent, an acid catalyst, reaction temperature, reaction time, each use amount) similar to those in the reaction [I].

Specific examples of the compound represented by the general formula (33) includes the same one as the specific examples of the compound represented by the general formula (32), and the preferable ones are also the same.

In the case where the compound represented by the general formula (32) and the compound represented by the general formula (33) are the same compound, in the reactions [I] and [II], the compound represented by the general formula (34) may be obtained in one-time reaction operation by the addition of the compound represented by the general formula (32) and the compound represented by the general formula (33) simultaneously. In this case, use amount of each of the compound represented by the general formula (32) and the compound represented by the general formula (33) is the same as the above-described use amount of the compound represented by the general formula (32), and preferable use amount is also the same. In addition, regarding reaction conditions (a reaction solvent, an acid catalyst, reaction temperature, reaction time, each use amount), they may be reacted under the reaction conditions similar to those in the reaction [I].

In the reaction [III], the compound represented by general formula (34) and the compound represented by the following general formula (35) may be reacted in a solvent, in the presence of a dehydration condensation agent, usually at 0 to 80°C, preferably at 10 to 50°C, for usually 1 to 24 hours, and preferably 3 to 18 hours.

The solvent includes ethers such as diethyl ether, diisopropyl ether, ethyl methyl ether, tetrahydrofuran and 1,4-dioxane, dimethoxyethane; ketones such as acetone, dimethyl ketone, methyl ethyl ketone, diethyl ketone, 2-hexanone, tert-butyl methyl ketone, cyclopentanone and cyclohexanone; halogenated hydrocarbons such as chloromethane, chloroform, dichloromethane, dichloroethane, trichloroethane, carbon tetrachloride and chlorobenzene; hydrocarbons such as n-hexane, benzene, toluene and xylene; esters such as ethyl acetate, butyl acetate and methyl propionate; nitriles such as acetonitrile; amides such as N,N-dimethylformamide; and the like. Among them, the ethers, the halogenated hydrocarbons and the hydrocarbons are preferable, and tetrahydrofuran, dichloromethane and toluene are more preferable. They may be used singly, or in combination of two or more kinds thereof as appropriate. Use amount of the reaction solvent is usually 1 to 50 times, and preferably 5 to 10 times, relative to total weight of the compound represented by the general formula (34) and the compound represented by the general formula (35).

The dehydration condensation agent may be the one generally used as a dehydration condensation agent, and includes, for example, inorganic dehydrating agents such as diphosphorus pentaoxide and anhydrous zinc chloride; carbodiimides such as dicyclohexylcarbodiimide, diisopropylcarbodiimide and 1-ethyl-3-(3-dimethylaminopropylcarbodiimide) hydrochloride; polyphosphoric acid; acetic anhydride; sulfuric acid; carbonyldiimidazole; p-toluene sulfonic acid; and the like; and the carbodiimides are preferable. Use amount of the dehydration condensation agent is usually 1 to 20 equivalents, and preferably 1 to 10 equivalents, relative to mole number of the compound represented by the general formula (34). In the reaction [III], a catalyst, such as dimethylaminopyridine, may be used to enhance efficiency of the dehydration condensation agent. Use amount of the catalyst is usually 0.1 to 10 equivalents, relative to mole number of the compound represented by the general formula (34).

Use amount of the compound represented by the general formula (35) is usually 1 to 2 equivalents, and preferably 1 to 1.5 equivalents, relative to mole number of the compound represented by the general formula (34).

Preferable specific examples of the compound represented by the general formula (35) include, for example, the following ones.

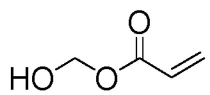 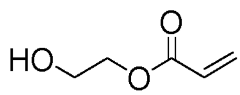 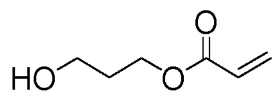

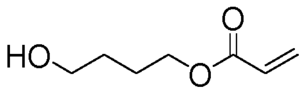
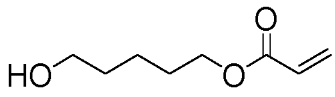
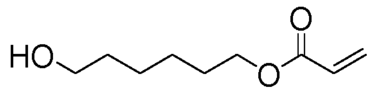

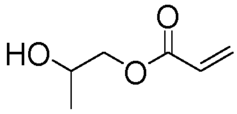
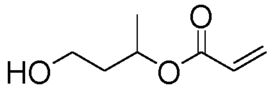
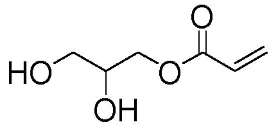

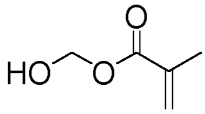
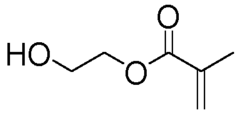
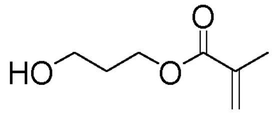

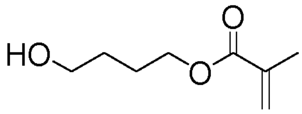
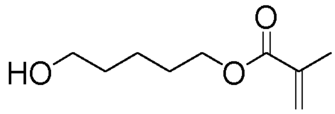
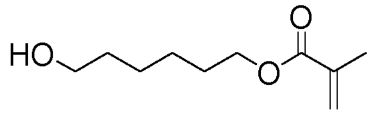

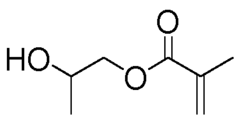
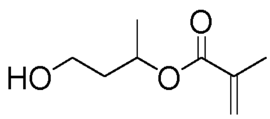
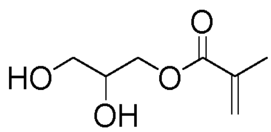

[Oxidation reaction]

The oxidation reaction in the reaction [IV] is carried out by reacting the triphenylmethane-based compound represented by the general formula (36) in a solvent, in the presence of an oxidant, usually at 0 to 80°C, preferably at 10 to 50°C, for usually 1 to 36 hours, and preferably 6 to 24 hours.

The oxidant includes, for example, an organic oxidant such as chloranil, dichlorodicyanobenzoquinone and N-2,4,6-trinitrophenyl-N',N'-phenyldihydrazine; an inorganic oxidant such as lead dioxide, manganese dioxide, potassium permanganate, potassium chromate and selenium dioxide; and the like. In addition, the reaction can be carried out also in a system of combination of chloranil, a metallic complex and hydrogen peroxide. A quinone-based oxidant is preferable in usability, and among them, chloranil is preferable. Use amount of the oxidant is usually 1 to 5 equivalents, and preferably 1 to 2 equivalents, relative to mole number of the triphenylmethane-based compound represented by the general formula (36).

It is preferable that the oxidation reaction is carried out through a chloride salt by the coexistence with hydrochloric acid. Use amount of the hydrochloric acid is usually 1 to 50 equivalents, and preferably 1 to 10 equivalents, relative to mole number of the triphenylmethane-based compound represented by the general formula (36).

The solvent used in the oxidation reaction includes the same one as the solvent used in the reaction [III], and the preferable one is also the same. Use amount of the reaction solvent is usually 1 to 100 times, and preferably 30 to 70 times, relative to total weight of the triphenylmethane-based compound represented by the general formula (36).

[Salt exchange reaction]

The salt exchange reaction in the reaction [IV] is carried out by contacting a salt of the anion of the present invention, in a solvent, with the triphenylmethane-based compound represented by the general formula (36), after the oxidation reaction.

The salt exchange reaction is carried out usually at 0 to 80°C, preferably at 10 to 50°C, for usually 1 to 24 hours, and preferably 1 to 8 hours.

The salt of the anion of the present invention in the salt exchange reaction includes a sodium salt, a potassium salt, a lithium salt, and the like, of the anion of the present invention; and a potassium salt or a lithium salt is preferable. Use amount of the salt of the anion of the present invention is usually 1 to 2 equivalents, and preferably 1 to 1.5 equivalents, relative to mole number of the triphenylmethane-based compound represented by the general formula (36) after the oxidation reaction.

In the reaction [IV], the oxidation reaction and the salt exchange reaction may be carried out sequentially as a

one-step reaction. In this case, after the oxidation reaction, the reaction may be carried out by the addition of the salt of the anion of the present invention to the reaction solution, in an amount of the above-described range, at the temperature and for the time in the salt exchange reaction.

Pressure in the reactions [I] to [IV] is not especially limited, as long as a series of the reactions is carried out without delay, and the reactions may be carried out, for example, under an ambient pressure.

The resulting reactants and products obtained after the reactions [I] to [IV] may be isolated, as needed, by a general post-treatment operation and purification operation usually carried out in this field. Specifically, for example, the resulting reactants and products may be isolated by filtration, washing, extraction, concentration under reduced pressure, recrystallization, distillation, column chromatography, or the like.

[Polymer of the present invention]

The polymer of the present invention is a polymer having a monomer unit derived from the compound of the present invention.

Weight average molecular weight (Mw) of the polymer of the present invention is usually 2,000 to 100,000, and preferably 2,000 to 50,000, and more preferably 2,000 to 30,000. In addition, distribution degree thereof (Mw/Mn) is usually 1.00 to 5.00, and preferably 1.00 to 3.00.

The polymer of the present invention may be a homopolymer or a copolymer, as long as it is the one having the monomer unit derived from the compound represented by the general formula (1), and the copolymer having high heat resistance effect is preferable.

The copolymer includes, for example, the one having one to two kinds of monomer units derived from the compound represented by the following general formula (2), the general formula (3), the general formula (4) or the general formula (5), and the monomer unit derived from the compound represented by the general formula (1), as configuration components:

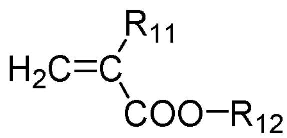 (2)

[wherein $R_{11}$ represents a hydrogen atom or a methyl group; $R_{12}$ represents a hydrogen atom, an alkyl group having 1 to 18 carbon atoms, a hydroxyalkyl group having 1 to 10 carbon atoms, an aryl group having 6 to 10 carbon atoms, an arylalkyl group having 7 to 13 carbon atoms, an alkoxyalkyl group having 2 to 9 carbon atoms, an alkoxyalkoxyalkyl group having 3 to 9 carbon atoms, an aryloxyalkyl group having 7 to 13 carbon atoms, a morpholinoalkyl group having 5 to 7 carbon atoms, a trialkylsilyl group having 3 to 9 carbon atoms, an alicyclic hydrocarbon group having 6 to 12 carbon atoms which has an oxygen atom or no oxygen atom, a dialkylaminoalkyl group having 3 to 9 carbon atoms, a fluoroalkyl group having 1 to 18 carbon atoms, an N-alkylenephthalimide group having 9 to 14 carbon atoms, a group represented by the following general formula (2-1):

$$-\left(R_{21}-O\right)_q-R_{22} \qquad (2\text{-}1)$$

(wherein $R_{21}$ represents an alkylene group having 1 to 3 carbon atoms, which has a hydroxy group as a substituent or no substituent; $R_{22}$ represents a phenyl group, which has a hydroxy group as a substituent or no substituent, or an alkyl group having 1 to 3 carbon atoms; and q represents an integer of 1 to 3.), a group represented by the following general formula (2-2):

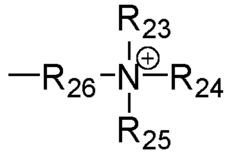 (2-2)

(wherein $R_{23}$ to $R_{25}$ represent an alkyl group having 1 to 3 carbon atoms; $R_{26}$ represents an alkylene group having 1 to 3 carbon atoms.),

or a group represented by the following general formula (2-3):

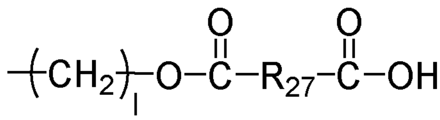 (2-3)

(wherein 1 represents an integer of 1 to 6; and $R_{27}$ represents a phenylene group or a cyclohexylene group.)]

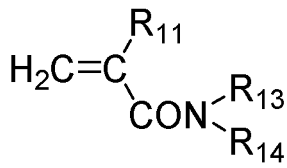 (3)

(wherein $R_{11}$ is the same as described above; $R_{13}$ represents a hydrogen atom, or an alkyl group having 1 to 3 carbon atoms; $R_{14}$ represents a hydrogen atom, an alkyl group having 1 to 3 carbon atoms, a dialkylaminoalkyl group having 3 to 9 carbon atoms, or a hydroxyalkyl group having 1 to 6 carbon atoms; and $R_{13}$ and $R_{14}$ may form a morpholino group together with a nitrogen atom adjacent thereto.)

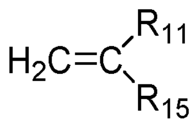 (4)

(wherein $R_{15}$ represents a phenyl group or a pyrrolidino group; and $R_{11}$ is the same as described above.)

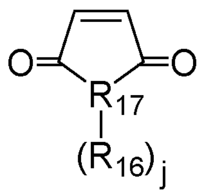 (5)

(wherein $R_{17}$ represents a nitrogen atom or an oxygen atom; represents 0 when $R_{17}$ is an oxygen atom, and 1 when $R_{17}$ is a nitrogen atom; $R_{16}$ represents a hydrogen atom, an alkyl group having 1 to 20 carbon atoms, a hydroxyalkyl group having 1 to 10 carbon atoms, a halogenated alkyl group having 1 to 10 carbon atoms, an alkylcycloalkyl group having 6 to 10 carbon atoms, a halogenated cycloalkyl group having 6 to 7 carbon atoms, an aryl group having 6 to 10 carbon atoms, an aryl group having 6 to 10 carbon atoms, which has an alkyl group having 1 to 6 carbon atoms as a substituent, or a halogenated aryl group having 6 to 10 carbon atoms.)

As $R_{11}$ in the general formula (2), a methyl group is preferable.

The alkyl group having 1 to 18 carbon atoms in $R_{12}$ of the general formula (2) may be any of the linear, branched or cyclic one, specifically including, for example, a methyl group, an ethyl group, an n-propyl group, an isopropyl group, an n-butyl group, an isobutyl group, a sec-butyl group, a tert-butyl group, an n-pentyl group, an isopentyl group, a sec-pentyl group, a tert-pentyl group, a neopentyl group, an n-hexyl group, an isohexyl group, a sec-hexyl group, a tert-hexyl group, a 3-methylpentyl group, a 2-methylpentyl group, a 1,2-dimethylbutyl group, an n-heptyl group, an isoheptyl group, a sec-heptyl group, an n-octyl group, an isooctyl group, a sec-octyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, a cyclopropyl group, a cyclopentyl group, a cyclohexyl group, a cycloheptyl group, a cyclooctyl group, a cyclodecyl group, a cycloundecyl group, a cyclododecyl group, a cyclotetradecyl group, a cyclooctadecyl group, and the like, and a methyl group and an ethyl group are preferable.

The hydroxyalkyl group having 1 to 10 carbon atoms in $R_{12}$ of the general formula (2) includes, for example, a hydroxymethyl group, a hydroxyethyl group, a hydroxypropyl group, a hydroxybutyl group, a hydroxypentyl group, a hydroxyhexyl group, a hydroxyheptyl group, a hydroxyoctyl group, a hydroxynonyl group, a hydroxydecyl group, and the like.

The aryl group having 6 to 10 carbon atoms in $R_{12}$ of the general formula (2) includes a phenyl group, a naphthyl group, and the like.

The arylalkyl group having 7 to 13 carbon atoms in $R_{12}$ of the general formula (2) includes, for example, a

benzyl group, a phenylethyl group, a phenylpropyl group, a naphthylmethyl group, a naphthylethyl group, a naphthylpropyl group, and the like, and a benzyl group is preferable.

The alkoxyalkyl group having 2 to 9 carbon atoms in $R_{12}$ of the general formula (2) includes, for example, a methoxymethyl group, a methoxyethyl group, a methoxypropyl group, a methoxybutyl group, a methoxypentyl group, a methoxyhexyl group, a methoxyheptyl group, a methoxyoctyl group, an ethoxymethyl group, an ethoxyethyl group, an ethoxypropyl group, an ethoxybutyl group, an ethoxypentyl group, an ethoxyhexyl group, an ethoxyheptyl group, a propoxymethyl group, a propoxyethyl group, a propoxypropyl group, a propoxybutyl group, a propoxypentyl group, a propoxyhexyl group, and the like.

The alkoxyalkoxyalkyl group having 3 to 9 carbon atoms in $R_{12}$ of the general formula (2) includes, for example, a methoxymethoxymethyl group, a methoxymethoxyethyl group, a methoxymethoxypropyl group, an ethoxymethoxymethyl group, an ethoxymethoxyethyl group, an ethoxymethoxypropyl group, a propoxymethoxymethyl group, a propoxymethoxyethyl group, a propoxymethoxypropyl group, an ethoxyethoxymethyl group, an ethoxyethoxyethyl group, an ethoxyethoxypropyl group, a propoxyethoxymethyl group, a propoxyethoxyethyl group, a propoxyethoxypropyl group, a propoxypropoxymethyl group, a propoxypropoxyethyl group, a propoxypropoxypropyl group, and the like.

The aryloxyalkyl group having 7 to 13 carbon atoms in $R_{12}$ of the general formula (2) includes, for example, a phenoxymethyl group, a phenoxyethyl group, a phenoxypropyl group, a naphthyloxymethyl group, a naphthyloxyethyl group, a naphthyloxypropyl group, and the like.

The morpholinoalkyl group having 5 to 7 carbon atoms in $R_{12}$ of the general formula (2) includes, for example, a morpholinomethyl group, a morpholinoethyl group, a morpholinopropyl group, and the like.

The trialkylsilyl group having 3 to 9 carbon atoms in $R_{12}$ of the general formula (2) includes, for example, a trimethylsilyl group, a triethylsilyl group, a tripropylsilyl group, a dimethylethylsilyl group, a diethylmethylsilyl group, and the like.

The alicyclic hydrocarbon group having 6 to 12 carbon atoms which has an oxygen atom, in $R_{12}$ of the general formula (2), includes, for example, a dicyclopentenyloxyethyl group, and the like.

The alicyclic hydrocarbon group having 6 to 12 carbon atoms which has no oxygen atom, in $R_{12}$ of the general formula (2), includes, for example, a cyclohexyl group, an isobornyl group, a dicyclopentanyl group, and the like.

The dialkylaminoalkyl group having 3 to 9 carbon atoms in $R_{12}$ of the general formula (2) includes, for example, a dimethylaminomethyl group, a dimethylaminoethyl group, a dimethylaminopropyl group, a diethylaminomethyl group, a diethylaminoethyl group, a diethylaminopropyl group, a dipropylaminomethyl group, a dipropylaminoethyl group, a dipropylaminopropyl group, and the like.

The fluoroalkyl group having 1 to 18 carbon atoms in $R_{12}$ of the general formula (2) includes, for example, a 2,2,2-trifluoroethyl group, a 2,2,3,3-tetrafluoropropyl group, a 2,2,3,3,4,4-hexafluorobutyl group, a 2,2,3,3,4,4,5,5-octafluoropentyl group, a 3,3,4,4,5,5,6,6,7,7,8,8,9,9,10,10,10-heptadecafluorodecyl group, a 2-(heptadecafluorooctyl)ethyl group, and the like.

The N-alkylenephthalimide group having 9 to 14 carbon atoms in $R_{12}$ of the general formula (2) includes, for example, a 2-phthalimideethyl group, a 2-tetrahydrophthalimideethyl group, and the like.

The alkylene group having 1 to 3 carbon atoms, which has a hydroxy group as substituent or no substituent, in $R_{21}$ of the general formula (2-1), includes a methylene group, an ethylene group, a propylene group, a hydroxymethylene group, a hydroxyethylene group, a 1-hydroxypropylene group, a 2-hydroxypropylene group, and the like, and an ethylene group, a propylene group and a 2-hydroxypropylene group are preferable.

The phenyl group, which has a hydroxy group as a substituent or no substituent, in $R_{22}$ of the general formula (2-1), includes a hydroxyphenyl group, a phenyl group, and the like.

The alkyl group having 1 to 3 carbon atoms in $R_{22}$ of the general formula (2-1) includes a methyl group, an ethyl group, a propyl group, and the like.

Specific examples of the group represented by the general formula (2-1) include, a (4-hydroxyphenoxy)methyl group, a (4-hydroxyphenoxy)ethyl group, a (4-hydroxyphenoxy)propyl group, a 1-hydroxy-1-phenoxymethyl group, a 1-hydroxy-2-phenoxyethyl group, a 2-hydroxy-3-phenoxypropyl group, a methyltrimethylene glycol group, a methyltriethylene glycol group, a methyltripropylene glycol group, and the like. Among them, a (4-hydroxyphenoxy)propyl group, a 2-hydroxy-3-phenoxypropyl group, a methyltripropylene glycol group and a methyltriethylene glycol group are preferable.

The alkyl group having 1 to 3 carbon atoms in $R_{23}$ to $R_{25}$ of the general formula (2-2) includes a methyl group, an ethyl group, a propyl group, and the like, and a methyl group is preferable.

The alkylene group having 1 to 3 carbon atoms in $R_{26}$ of the general formula (2-2) includes a methylene group, an ethylene group, a propylene group, and the like.

Specific examples of the group represented by the general formula (2-2) include a trimethylammoniummethyl group, a trimethylammoniumethyl group, a triethylammoniummethyl group, a triethylammoniumethyl group, and the like.

Preferable specific examples of the group represented by the general formula (2-3) include, for example, the following ones.

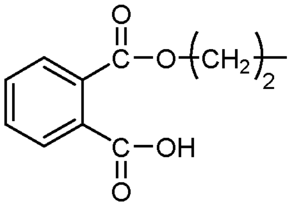
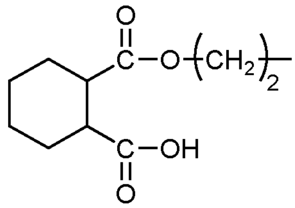

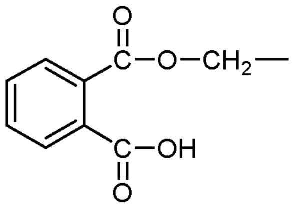
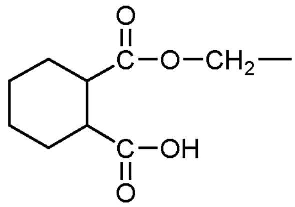

As $R_{12}$ in the general formula (2), a hydrogen atom, an alkyl group having 1 to 18 carbon atoms, a hydroxyalkyl group having 1 to 10 carbon atoms, an aryl group having 6 to 10 carbon atoms, an arylalkyl group having 7 to 13 carbon atoms, an alkoxyalkyl group having 2 to 9 carbon atoms, an aryloxyalkyl group having 7 to 13 carbon atoms, the group represented by the general formula (2-1), and the group represented by the general formula (2-3) are preferable. Among them, a hydrogen atom, an alkyl group having 1 to 18 carbon atoms, a hydroxyalkyl group having 1 to 10 carbon atoms, an aryl group having 6 to 10 carbon atoms, an arylalkyl group having 7 to 13 carbon atoms, and an alkoxyalkyl group having 2 to 9 carbon atoms are more preferable, and a hydrogen atom, and an arylalkyl group having 7 to 13 carbon atoms are particularly preferable.

Preferable specific examples of the general formula (2) include acrylic acid, benzyl acrylate, methacrylic acid, benzyl methacrylate, hydroxyethyl methacrylate, methyl methacrylate, and the like. Among them, acrylic acid, benzyl acrylate, methacrylic acid and benzyl methacrylate are preferable, and methacrylic acid and benzyl methacrylate are more preferable.

The alkyl group having 1 to 3 carbon atoms in $R_{13}$ and $R_{14}$ of the general formula (3) includes a methyl group, an ethyl group, a propyl group, and the like.

The dialkylaminoalkyl group having 3 to 9 carbon atoms in $R_{14}$ of the general formula (3) includes a dimethylaminomethyl group, a dimethylaminoethyl group, a dimethylaminopropyl group, a diethylaminomethyl group, a diethylaminoethyl group, a diethylaminopropyl group, a dipropylaminomethyl group, a dipropylaminoethyl group, a dipropylaminopropyl group, and the like.

The hydroxyalkyl group having 1 to 6 carbon atoms in $R_{14}$ of the general formula (3) includes a hydroxymethyl group, a hydroxyethyl group, a hydroxypropyl group, a hydroxybutyl group, a hydroxypentyl group, a hydroxyhexyl group, and the like, and a hydroxyethyl group is preferable.

Preferable specific examples of the general formula (3) include (meth)acrylamide, N,N-dimethylacrylamide, N,N-diethylacrylamide, hydroxyethyl (meth)acrylamide, 4-acryloyl morpholine, and the like. Among them, (meth)acrylamide, N,N-dimethylacrylamide and N,N-diethyl acrylamide are preferable, and N,N-diethylacrylamide is particularly preferable.

Preferable specific examples of the general formula (4) include styrene, α-methylstyrene, N-vinylpyrrolidone, and the like. Among them, styrene and α-methylstyrene are preferable, and styrene is particularly preferable.

The alkyl group having 1 to 20 carbon atoms in $R_{16}$ of the general formula (5) may be any of the linear, branched and cyclic one, specifically including, for example, a methyl group, an ethyl group, an n-propyl group, an isopropyl group, an n-butyl group, an isobutyl group, a tert-butyl group, a 1-methylpropyl group, an n-pentyl group, an isopentyl group, a tert-pentyl group, a neopentyl group, a 1-methylbutyl group, an n-hexyl group, an isohexyl group, a tert-hexyl group, a 3-methylpentyl group, a 2-methylpentyl group, a 1-methylpentyl group, a 1,2-dimethylbutyl group, a cyclohexyl group, an n-heptyl group, an isoheptyl group, a 1-methylhexyl group, an n-octyl group, an isooctyl group, a 1-methylheptyl group, a 2-ethylhexyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, a nonadecyl group, an icosyl group, and the like.

The hydroxyalkyl group having 1 to 10 carbon atoms in $R_{16}$ of the general formula (5) includes, for example, a hydroxymethyl group, a hydroxyethyl group, a hydroxypropyl group, a hydroxybutyl group, a hydroxypentyl group, a hydroxyhexyl group, a hydroxyheptyl group, a hydroxyoctyl group, a hydroxynonyl group, a hydroxydecyl group, and the like.

The halogenated alkyl group having 1 to 10 carbon atoms in $R_{16}$ of the general formula (5) includes, for example,

a chloromethyl group, a chloroethyl group, a chloro-n-propyl group, a chloroisopropyl group, a chloro-n-butyl group, a chloro-tert-butyl group, a chloro-n-pentyl group, a chloro-n-hexyl group, a chloro-n-heptyl group, a chloro-n-octyl group, a chloro-n-nonyl group, a chloro-n-decyl group, a fluoromethyl group, a fluoroethyl group, a fluoro-n-propyl group, a fluoroisopropyl group, a fluoro-n-butyl group, a fluoro-tert-butyl group, a fluoro-n-pentyl group, a fluoro-n-hexyl group, a fluoro-n-heptyl group, a fluoro-n-octyl group, a fluoro-n-nonyl group, a fluoro-n-decyl group, and the like.

The alkylcycloalkyl group having 6 to 10 carbon atoms in $R_{16}$ of the general formula (5) includes, for example, a methylcyclopentyl group, an ethylcyclopentyl group, a propylcyclopentyl group, a methylcyclohexyl group, an ethylcyclohexyl group, a propylcyclohexyl group, a butylcyclohexyl group, a methylcycloheptyl group, an ethylcycloheptyl group, a propylcycloheptyl group, a methylcyclooctyl group, an ethylcyclooctyl group, and the like.

The halogenated cycloalkyl group having 6 to 7 carbon atoms in $R_{16}$ of the general formula (5) includes, for example, a chlorocyclohexyl group, a fluorocyclohexyl group, a bromocyclohexyl group, a chlorocycloheptyl group, a fluorocycloheptyl group, a bromocycloheptyl group, and the like.

The aryl group having 6 to 10 carbon atoms in $R_{16}$ of the general formula (5) includes a phenyl group, a naphthyl group, and the like.

The aryl group having 6 to 10 carbon atoms, which has an alkyl group having 1 to 6 carbon atoms as a substituent, in $R_{16}$ of the general formula (5), includes, for example, a methylphenyl group, an ethylphenyl group, an n-propylphenyl group, an n-butylphenyl group, an n-pentylphenyl group, an n-hexylphenyl group, a methylnaphthyl group, an ethylnaphthyl group, an n-propylnaphthyl group, and the like.

The halogenated aryl group having 6 to 10 carbon atoms in $R_{16}$ of the general formula (5) includes, for example, a chlorophenyl group, a fluorophenyl group, a chloronaphthyl group, a fluoronaphthyl group, and the like.

Preferable specific examples of the general formula (5) include maleic anhydride, maleimide, N-methylmaleimide, N-ethylmaleimide, N-butylmaleimide, N-octylmaleimide, N-dodecylmaleimide, N-(2-ethylhexyl)maleimide, N-(2-hydroxyethyl)maleimide, N-(2-chlorohexyl)maleimide, N-cyclohexylmaleimide, N-(2-methylcyclohexyl)maleimide, N-(2-ethylcyclohexyl)maleimide, N-(2-chlorocyclohexyl)maleimide, N-phenylmaleimide, N-(2-methylphenyl)maleimide, N-(2-ethylphenyl)maleimide, N-(2-chlorophenyl)maleimide, and the like, and among them, N-phenylmaleimide is preferable.

The copolymer of the present invention specifically includes those having monomer unit combinations described in the following table, and among them, the combinations 1, 5, 6, and 7 are preferable. In addition, in the following combination 1, a combination comprising the compound represented by the general formula (1) and two kinds of the compound represented by the general formula (2) is preferable.

| | Compound from which monomer unit is derived | | |
|---|---|---|---|
| Combination 1 | General formula (1) | General formula (2) | - |
| Combination 2 | General formula (1) | General formula (3) | - |
| Combination 3 | General formula (1) | General formula (4) | - |
| Combination 4 | General formula (1) | General formula (5) | - |
| Combination 5 | General formula (1) | General formula (2) | General formula (3) |
| Combination 6 | General formula (1) | General formula (2) | General formula (4) |
| Combination 7 | General formula (1) | General formula (2) | General formula (5) |

Weight ratio of the monomer unit derived from the compound represented by the general formula (1), and the monomer unit derived from the compound represented by the general formula (2), the general formula (3), the general formula (4), or the general formula (5) may be set as appropriate, depending on kinds of the monomer units to be used, however, the monomer unit derived from the compound represented by the general formula (1) is usually 1 to 90% by weight, and preferably 5 to 85% by weight, relative to total weight of the resulting polymer.

Preferable specific examples of the copolymer of the present invention include polymers comprising the monomer unit derived from the compound represented by the general formula (1), and one kind or two kinds of a monomer unit derived from a compound represented by the following general formula (2').

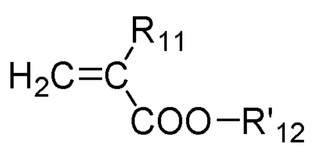

(2' )

(wherein $R_{11}$ is the same as described above; $R'_{12}$ represents a hydrogen atom, an alkyl group having 1 to 18 carbon atoms, a hydroxyalkyl group having 1 to 10 carbon atoms, an aryl group having 6 to 10 carbon atoms, an arylalkyl group having 7 to 13 carbon atoms, or an alkoxyalkyl group having 2 to 9 carbon atoms.)

Specific examples of the alkyl group having 1 to 18 carbon atoms, the hydroxyalkyl group having 1 to 10 carbon atoms, the aryl group having 6 to 10 carbon atoms, the arylalkyl group having 7 to 13 carbon atoms, and the alkoxyalkyl group having 2 to 9 carbon atoms, in $R'_{12}$ of the general formula (2'), include the same ones as those of $R_{12}$ in the general formula (2).

As $R'_{12}$ in the general formula (2'), a hydrogen atom, an alkyl group having 1 to 18 carbon atoms, and an arylalkyl group having 7 to 13 carbon atoms are preferable, and a hydrogen atom, and the arylalkyl group having 7 to 13 carbon atoms are more preferable.

Preferable specific examples of the general formula (2') include acrylic acid, benzyl acrylate, methacrylic acid, benzyl methacrylate, and the like, and among them, methacrylic acid and benzyl methacrylate are preferable.

[Production method for the polymer of the present invention]

The polymer of the present invention is produced, for example, as follows. That is, the polymer of the present invention can be obtained by subjecting the compound of the present invention obtained as described above to a polymerization reaction known per se. When the polymer of the present invention is a copolymer, in the polymerization reaction, polymerization may be carried out after mixing the compound of the present invention with one to two kinds of the compound represented by the general formula (2), the general formula (3), the general formula (4) or the general formula (5), so that ratio of the monomer unit derived from each monomer in the finally obtained polymer attains as described above.

The polymerization reaction is carried out, for example, as follows. That is, the compound represented by the general formula (1) having the anion of the present invention, or the compound represented by the general formula (1) having the anion of the present invention, and one to two kinds of the compound represented by the general formula (2), the general formula (3), the general formula (4) or the general formula (5) are dissolved in 1 to 10 times volume of an appropriate solvent such as toluene, 1,4-dioxane, tetrahydrofuran, isopropanol, methyl ethyl ketone and propylene glycol monomethyl ether acetate, relative to total volume thereof. Then, in the presence of 0.01 to 30% by weight of a polymerization initiator such as azoisobutyronitrile, 2,2'-azobis(2,4-dimethylvaleronitrile), 2,2'-azobis(2-methylpropionate), 2,2'-azobis(2-methylbutyronitrile), benzoylperoxide and lauroyl peroxide, relative to total amount of the dissolved compounds; a reaction may be carried out at 50 to 150 °C for 1 to 48 hours. After the reaction, treatment may also be carried out according to a conventional method for polymer acquisition.

[Colored composition]

The colored composition of the present invention is the one containing at least one kind of the compound or the polymer of the present invention. The colored composition is the one having less fading caused by heating, and still more, it is capable of forming an excellent colored cured film having heat resistance. Therefore, it can be used in an application of formation of a colored pixel such as a color filter used in a liquid crystal display (LCD) or a solid-state imaging element (CCD, CMOS, or the like), or in applications of printing ink, inkjet ink, paint, and the like; and particularly, it can be suitably used for the color filter of the liquid crystal display. Still more, the colored composition of the present invention can also be used as a colored resin molded product by molding to a sheet, a film, a bottle, a cup, or the like, using a conventionally known molding method. Accordingly, it can also be used in applications of spectacles, contact lens, color contact lens, or the like; and it can be used in similar applications also by making a multi-layered structure with a known resin. In addition, it can also be used in applications of, for example, an optical film, a hair coloring agent, a labeling material for a compound or a biological material, a material of an organic solar battery, or the like. The colored composition of the present invention may contain an additive used in this field usually, and the like, depending on each application, besides the compound or the polymer of the present invention.

For example, in the case of using the colored composition of the present invention as a colored resin, the colored composition of the present invention is preferably the one which contains at least one or more kinds of the compound or the polymer of the present invention, as well as which is mixed with other resins, and more preferably the one which has one or more kinds of the polymer of the present invention and is mixed with other resins. The other resins are not especially limited, and include, for example, a polyolefin resin, a polystyrene resin, a polyester resin, a polyamide resin, a polyurethane resin, a polycarbonate resin, an epoxy resin, an acrylic resin, an acrylonitrile resin, an ABS resin, and the like. More specifically, a homopolymer or a copolymer derived from the compound represented by the general formula (2), the general formula (3), the general formula (4) and/or the general formula (5) is preferable, and the homopolymer derived from the compound represented by the general formula (2), the general formula (3), the general formula (4) or the general formula (5) is more preferable. As the homopolymer, the homopolymer derived from the compound

represented by the general formula (2) is preferable, and the homopolymer derived from the compound represented by the general formula (2') is more preferable. In addition, in the case of mixing with the other resins, mixing ratio thereof may be set appropriately depending on required color of the colored resin. In the case of using the colored composition of the present invention as the colored resin, it may be used after molding it by a molding method known per se. Further, the colored composition of the present invention may contain an additive usually used in this field, such as a lubricant, an antistatic agent, a UV inhibitor, an antioxidant, a light stabilizer, a dispersing agent, a processing stabilizer, a processing aid, an impact modifier, fillers, a reinforcing agent, a flame-proofing agent, a plasticizer and a foaming agent; besides the compound or the polymer of the present invention and, if necessary, the other resins; within a range not interfering with the objects and effects of the present invention. The colored composition of the present invention has less elution of a dye even in contact with a solvent and excellent weather resistance, in the case of using as the colored resin applications.

For example, in the case of using the colored composition of the present invention in colored pixel formation, the colored composition of the present invention is preferably the one containing at least one or more kinds of the compound or the polymer of the present invention, and a polymerization initiator, a binder resin, as well as a radically polymerizable monomer or oligomer, and if necessary, which may contain a pigment, a solvent, a silane coupling agent and a cross-linking agent, and the like. The colored composition contains 1 to 50%, and preferably 5 to 30% of the compound or the polymer of the present invention, relative to weight of the colored composition. It should be noted that, weight of the colored composition referred to herein means weight of solid components excluding a solvent, and means the same hereafter in the present application.

As the polymerization initiator, a known thermal polymerization initiator or a photo polymerization initiator can be used, and a photo polymerization initiator is preferable. Specifically, it includes an acetophenone-type polymerization initiator such as diethoxyacetophenone, 2-hydroxy-2-methyl-1-phenylpropane-1-one, benzyldimethylketal, 1-(4-isopropylphenyl)-2-hydroxy-2-methylpropane-1-one, 4-(2-hydroxyethoxy)phenyl-(2-hydroxy-2-propyl)ketone, 1-hydroxycyclohexyl-phenylketone, 2-methyl-2-morpholino(4-thiomethylphenyl)propane-1-one and 2-benzyl-2-dimethylamino-1-(4-morpholinophenyl)-butanone; a benzoin-type polymerization initiator such as benzoin, benzoin isopropyl ether and benzoin isobutyl ether; an acyl phosphine oxide-type polymerization initiator such as 2,4,6-trimethylbenzoyldiphenyl phosphine oxide; a benzyl, methyl phenylglyoxylate-type polymerization initiator; a benzophenone-type polymerization initiator such as benzophenone, methyl o-benzoylbenzoate, 4-phenylbenzophenone, 4,4'-dichlorobenzophenone, hydroxybenzophenone, 4-benzoyl-4'-methyl-diphenylsulfide, acrylated benzophenone, 3,3',4,4'-tetra(tert-butylperoxycarbonyl)benzophenone and 3,3'-dimethyl-4-methoxybenzophenone; a thioxanthone-type polymerization initiator such as 2-isopropylthioxanthone, 2,4-dimethylthioxanthone, 2,4-diethylthioxanthone and 2,4-dichlorothioxanthone; an aminobenzophenone-type polymerization initiator such as Michler's ketone and 4,4'-diethylaminobenzophenone; an oxime ester-type polymerization initiator such as 1-[4-(phenylthio)phenyl]-1,2-octanedione-2-(o-benzoyloxime) and 1-[6-(2-methylbenzoyl)-9-ethyl-9H-carbazol-3-yl]ethanone-o-acetyloxime; 10-butyl-2-chloroacridone, 2-ethylanthraquinone, 9,10-phenanthrenequinone, camphor quinone; and the like.

The polymerization initiator may be contained singly, or in two or more kinds. Content thereof is 1 to 50% by weight, and preferably 5 to 30% by weight, relative to weight of the colored composition.

The binder resin includes, for example, an ethylenically unsaturated monomer having at least one of a carboxy group or a hydroxy group; a copolymer of the ethylenically unsaturated monomer and an ethylenically unsaturated monomer having an aromatic hydrocarbon group or an aliphatic hydrocarbon group; the one having an epoxy group at the side chain or the terminal, or the like, of the copolymer; the one to which an acrylate is added; and the like. They may be used singly, or in combination of two or more kinds.

Specific examples of the ethylenically unsaturated monomer having the carboxy group include unsaturated monocarboxylic acids such as acrylic acid, methacrylic acid, benzyl methacrylate, crotonic acid, $\alpha$-chloroacrylic acid, ethacrylic acid and cinnamic acid; unsaturated dicarboxylic acids (anhydrides) such as maleic acid, maleic anhydride, fumaric acid, itaconic acid, itaconic anhydride, citraconic acid, citraconic anhydride and mesaconic acid; tri or more polyvalent unsaturated carboxylic acids (anhydrides), 2-(meth)acryloyloxyethyl hexahydrophthalate, 2-methacryloyloxyethyl 2-hydroxypropylphthalate, 2-acryloyloxyethyl 2-hydroxyethylphthalate; and the like.

Content of the binder resin is 10% by weight to 50% by weight, and preferably 20% by weight to 50% by weight, relative to weight of the colored composition.

As an example, the radically polymerizable monomer or oligomer includes polyethylene glycol diacrylate (the one having 2 to 14 ethylene groups), polyethylene glycol dimethacrylate (the one having 2 to 14 ethylene groups), trimethylolpropane diacrylate, trimethylolpropane dimethacrylate, trimethylolpropane triacrylate, trimethylolpropane trimethacrylate, trimethylolpropane ethoxytriacrylate, trimethylolpropane ethoxytrimethacrylate, trimethylolpropane propoxytriacrylate, trimethylolpropane propoxytrimethacrylate, tetramethylolmethane triacrylate, tetramethylolmethane trimethacrylate, tetramethylolmethane tetraacrylate, tetramethylolmethane tetramethacrylate, polypropyleneglycol diacrylate (the one having 2 to 14 propylene groups), polypropylene glycol dimethacrylate (the one having 2 to 14 propylene groups), dipentaerythritol pentaacrylate, dipentaerythritol pentamethacrylate, dipentaerythritol hexaacrylate, dipentaerythritol hexamethacrylate, ethoxylated pentaerythritol tetraacrylate (the one having 40 or less ethoxy groups), propoxylated pentaerythritol tetraacrylate (the one having 40 or less propoxy groups), ethoxylated trimethylolpropane triacrylate (the one having 40 or less ethoxy groups), propoxylated trimethylolpropane triacrylate (the one having 40 or less propoxy groups), bisphenol A polyoxyethylene diacrylate, bisphenol A polyoxyethylene dimethacrylate, bisphenol A dioxyethylene diacrylate, bisphenol A dioxyethylene dimethacrylate, bisphenol A trioxyethylene diacrylate, bisphenol A trioxyethylene dimethacrylate, bisphenol A decaoxyethylene diacrylate, bisphenol A decaoxyethylene dimethacrylate, isocyanuric acid ethoxy modified triacrylate, an esterified product with a polyvalent carboxylic acid (phthalic anhydride, and the like) and a compound having a hydroxy group and an ethylenically unsaturated group (β-hydroxyethyl acrylate, β-hydroxymethyl methacrylate, and the like), an alkyl ester of acrylic acid or methacrylic acid (methyl acrylate, methyl methacrylate, ethyl acrylate, ethyl methacrylate, butyl acrylate, butyl methacrylate, 2-ethylhexyl acrylate, 2-ethylhexyl methacrylate, and the like), 2-hydroxyethyl acrylate, 2-hydroxyethyl methacrylate, phenoxyethyl acrylate, phenoxyethyl methacrylate, N,N-dimethylacrylamide, N,N-dimethylaminoethyl acrylate, a quaternary chloride of N,N-dimethylaminoethyl acrylate by methyl chloride, a quaternary chloride of N,N-dimethylaminopropylacrylamide by methyl chloride, acryloylmorpholine, N-isopropylacrylamide, N,N-diethylacrylamide, and the like. Among them, dipentaerythritol pentaacrylate, dipentaerythritol pentamethacrylate, dipentaerythritol hexaacrylate and dipentaerythritol hexamethacrylate are preferable, and dipentaerythritol pentaacrylate and dipentaerythritol hexaacrylate are more preferable.

The pigment may be a pigment that is used to prepare a colored pattern of blue color or green color, and for example, a phthalocyanine-based pigment, and the like, is included. The phthalocyanine-based pigment includes the one containing magnesium, titanium, iron, cobalt, nickel, copper, zinc or aluminum in central metal; specifically including C.I. pigment blue 15, C.I. pigment blue 15:1, C.I. pigment blue 15:2, C.I. pigment blue 15:3, C.I. pigment blue 15:4, C.I. pigment blue 15:5, C.I. pigment blue 15:6, C.I. pigment blue 16, C.I. pigment blue 17:1, C.I. pigment blue 75, C.I. pigment blue 79, C.I. pigment green 7, C.I. pigment green 36, C.I. pigment green 37, C.I. pigment green 58, chloroaluminum phthalocyanine, hydroxyaluminum phthalocyanine, aluminum phthalocyanine oxide and zinc phthalocyanine; and C.I. pigment blue 15, C.I. pigment blue 15:6, C.I. pigment blue 15:1, C.I. pigment blue 15:2 and C.I. pigment green 58 are preferable, and in particular, C.I. pigment blue 15:6 and C.I. pigment green 58 are preferable.

Content of the pigment is 10 to 50% by weight, and preferably 10 to 30% by weight, relative to weight of the colored composition.

In the case where the colored composition of the present invention contains the pigment, it is preferable to contain a pigment dispersant. The pigment dispersant includes, for example, polyamide amine and a salt thereof, polycarboxylic acid and a salt thereof, a high molecular weight unsaturated acid ester, modified polyurethane, modified polyester, modified poly(meth)acrylate, a (meth)acrylic copolymer, a naphthalene sulfonic acid/formalin condensate, a polyoxyethylene alkyl phosphoric acid ester, a polyoxyethylene alkylamine, an alkanol amine, and the like. The pigment dispersant may be used singly, or in combination of two or more kinds. Content thereof is usually 1 to 80% by weight, and preferably 10 to 60% by weight, relative to weight of the pigment.

The solvent may be appropriately selected depending on the components contained in the colored composition. Specifically, it includes, for example, ethyl acetate, n-butyl acetate, isobutyl acetate, amyl formate, isoamyl acetate, butyl propionate, isopropyl butyrate, ethyl butyrate, butyl butyrate, methyl lactate, ethyl lactate, methyl oxyacetate, ethyl oxyacetate, butyl oxyacetate, methyl methoxyacetate, ethyl methoxyacetate, butyl methoxyacetate, methyl ethoxyacetate, ethyl ethoxyacetate, methyl 3-oxypropionate, ethyl 3-oxypropionate, methyl 3-methoxypropionate, ethyl 3-methoxypropionate, methyl 3-ethoxypropionate, ethyl 3-ethoxypropionate, methyl 2-oxypropionate, ethyl 2-oxypropionate, propyl 2-oxypropionate, methyl 2-methoxypropionate, ethyl 2-methoxypropionate, propyl 2-methoxypropionate, methyl 2-ethoxypropionate, ethyl 2-ethoxypropionate, methyl 2-oxy-2-methylpropionate, methyl 2-methoxy-2-methylpropionate, ethyl 2-ethoxy-2-methylpropionate, methyl pyruvate, ethyl pyruvate, propyl pyruvate, methyl acetoacetate, ethyl acetoacetate, methyl 2-oxobutanoate, ethyl 2-oxobutaanoate, diethylene glycol dimethyl ether, tetrahydrofuran, ethylene glycol monomethyl ether, ethylene glycol monoethyl ether, methylcellosolve acetate, ethylcellosolve acetate, diethylene glycol monomethyl ether, diethylene glycol monoethyl ether, diethylene glycol monobutyl ether, propylene glycol monomethyl ether, propylene glycol monomethyl ether acetate, propylene glycol monoethyl ether acetate, propylene glycol monopropyl ether acetate, methyl ethyl ketone, cyclohexanone, 2-heptanone, 3-heptanone, and the like. Content of the solvent is an amount that concentration of the colored composition of the present invention attains 10% by weight to 80% by weight in the solvent.

The silane coupling agent is used in the case of bonding to a substrate, such as glass. As the silane coupling agent, a conventionally known one usually used in this field can be used, and it includes a silane coupling agent having, for example, an epoxy group, a thiol group, a hydroxy group, an amino group, an ureido group, a vinyl group, an acryloyl group, and the like, as a reactive organic functional group. Specifically, it includes β-(3,4-epoxycyclohexyl)ethyltrimethoxysilane, γ-glycidooxypropyltrimethoxysilane, γ-mercaptopropyltrimethoxysilane, γ-aminopropyltriethoxysilane, N-β-(aminoethyl)-γ-aminopropyltrimethoxysilane, γ-ureidopropyltriethoxysilane, vinyltriethoxysilane, vinyl-tris(β-methoxyethoxy)silane and γ-methacryloxypropyltrimethoxysilane. The silane coupling agent may be used usually in an amount of 0.1% by weight to 10% by weight, and preferably 1% by weight to 5% by weight, in the reaction solution.

The cross-linking agent is not especially limited as long as it is the one which is capable of carrying out film curing by a cross-linking reaction, and includes, for example, (a) an epoxy resin, (b) a melamine compound, a guanamine compound, a glycoluril compound or a urea compound, substituted with at least one substituent selected from a methylol group, an alkoxymethyl group and an acyloxymethyl group, and (c) a phenol compound, a naphthol compound or a hydroxyanthracene compound, substituted with at least one substituent selected from a methylol group, an alkoxymethyl group and an acyloxymethyl group; and among them, a polyfunctional epoxy resin is preferable.

Content of the cross-linking agent is 10% by weight to 50% by weight, and preferably 20% by weight to 50% by weight, relative to weight of the colored composition.

The colored composition of the present invention may contain a polymerization inhibitor, a surfactant, an additive, or the like, in addition to the above-described ones, and they are not especially limited, as long as they are those known per se, and the use amount is also not limited, as long as it is the amount usually used in this field.

The colored composition of the present invention is prepared by mixing with the above-described components.

The present invention is described below in further detail by Examples, however, the present invention should not be limited to these Examples.

## EXAMPLES

### Example 1

### Synthesis of a dye monomer 1

(1) Synthesis of a triphenylmethane derivative having a carboxyl group (a compound 3)

Into a round-bottom flask equipped with a stirring apparatus and a Dean-Stark apparatus, 5.0 g (33 mmol) of 4-formylbenzoic acid (a compound 1, produced by Wako Pure Chemical Industries, Ltd.), 16.1 g (133 mmol) of N,N-diethylaniline (a compound 2, produced by Wako Pure Chemical Industries, Ltd.), 60 ml of methyl isobutyl ketone (MIBK) (produced by Wako Pure Chemical Industries, Ltd.), and 6.3 g (33 mmol) of p-toluene sulfonic acid monohydrate (PTSA $H_2O$) (produced by Wako Pure Chemical Industries, Ltd.) were added, and refluxed for 11 hours. Dichloromethane and water were added thereto for extraction, and then an organic layer was collected by washing with water. The solvent was removed by concentration under reduced pressure from the organic layer to obtain green oil. Dichloromethane and 1 mol/L hydrochloric acid were added thereto for extraction to obtain a water layer, and then dichloromethane and 25% sodium hydroxide were added into the water layer for neutralization to collect an organic layer. Green oil, obtained by removing the solvent by concentration under reduced pressure, was purified by a silica-gel column chromatography, and the solvent was removed to obtain 9.3 g (yield: 65%) of a triphenylmethane derivative (a compound 3) as a green solid.

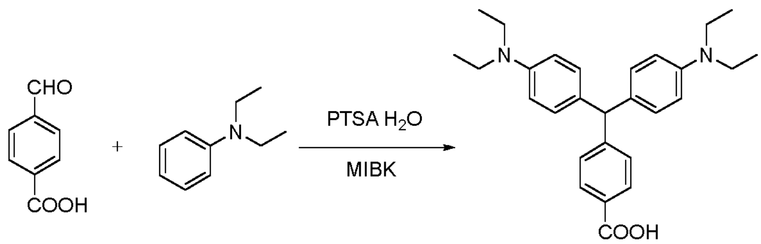


1 2 3

(2) Introduction of a polymerizable group

Into a round-bottom flask equipped with a stirring apparatus, 9.2 g (21.4 mmol) of the triphenylmethane derivative (the compound 3) obtained in (1), and 92 ml of dichloromethane were added and dissolved, and then 2.8 g (21.4 mmol) of 2-hydroxyethyl methacrylate (a compound 4, produced by Wako Pure Chemical Industries, Ltd.), 0.8 g (6.4 mmol) of 4-dimethylaminopyridine (DMAP) (produced by Wako Pure Chemical Industries, Ltd.), and 4.5 g (23.5 mol) of 1-ethyl-3-(3-dimethylaminopropyl)carbodiimide hydrochloride (WSC) (produced by Toyobo Co., Ltd.) were added and reacted at room temperature for 5 hours. Yellow oil, obtained by washing an organic layer with water and removing a solvent by concentration under reduced pressure, was purified by a silica-gel column chromatography to obtain 10.8 g (yield: 93%) of a triphenylmethane derivative (a compound 5) as yellow oil state.

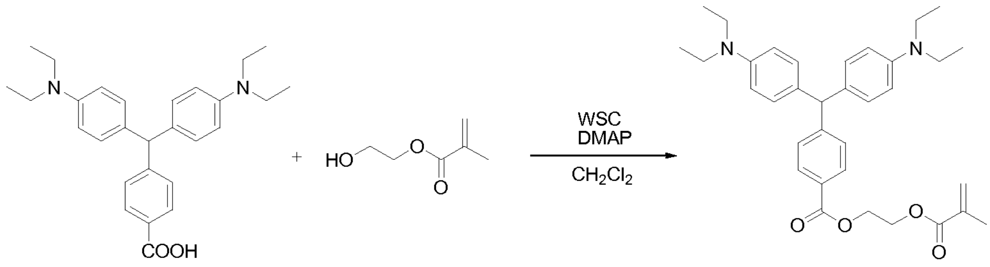


3 4 5

(3) An oxidation and salt exchange reaction

Into a round-bottom flask equipped with a stirring apparatus, 4.0 g (7.4 mmol) of the triphenylmethane derivative (the compound 5) obtained in (2), 80 ml of toluene, and 120 ml of dichloromethane were added and dissolved, and then 16 g of water and 1.5 g of concentrated hydrochloric acid were added and stirred at room temperature for 10 minutes. Into there, 1.8 g (7.3 mmol) of chloranil (produced by Wako Pure Chemical Industries, Ltd.) was added and stirred at room temperature for 1 hour, and then 6.0 g (7.2 mmol) of a lithium salt of tetrakis(pentafluorophenyl)boron(IV) (LiFABA) (produced by Tosoh Finechem Corp.) was added and reacted at room temperature for 16 hours. After completion of the reaction, an organic layer was obtained by the addition of dichloromethane and 1 mol/L hydrochloric acid, extraction and liquid separation. This organic layer was washed with water, a saturated aqueous solution of sodium hydrogen carbonate, and water in this order, and then a solvent was removed by concentration under reduced pressure. Dichloromethane was added thereto to remove insoluble substance, and the solvent was removed by concentration under reduced pressure and dried to obtain 7.2 g (yield: 79%) of a dye monomer 1 (a compound 6) as a green solid.

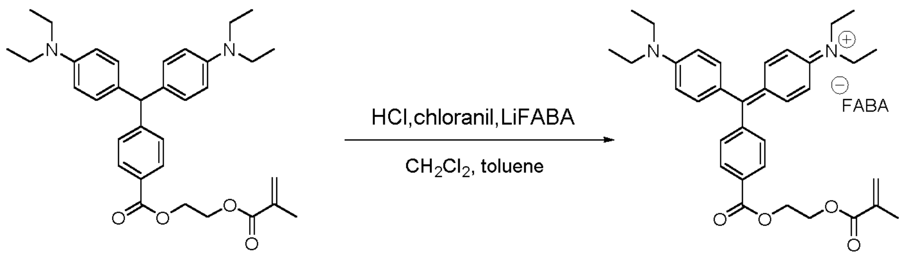


5 6

Comparative Example 1

Synthesis of a dye monomer 2

Into a round-bottom flask equipped with a stirring apparatus, 8.0 g (14.7 mmol) of the triphenylmethane derivative (the compound 5) obtained in (2), 160 ml of toluene and 160 ml of acetone were added and dissolved, and then 80 g of water, 3.0 g of concentrated hydrochloric acid, and 7.2 g (29.5 mmol) of chloranil (produced by Wako Pure Chemical Industries, Ltd.) were added and reacted at room temperature for 15 hours. After completion of the reaction, insoluble substance was filtrated, and an organic layer was obtained by the addition of dichloromethane and water, extraction and liquid separation. A green solid, obtained by removing a solvent from this organic layer by concentration under reduced pressure, was purified by a silica-gel column chromatography, and then a solvent was removed by concentration under reduced pressure and dried to obtain 7.7 g (yield: 91%) of a dye monomer 2 (a compound 7) as a green solid.

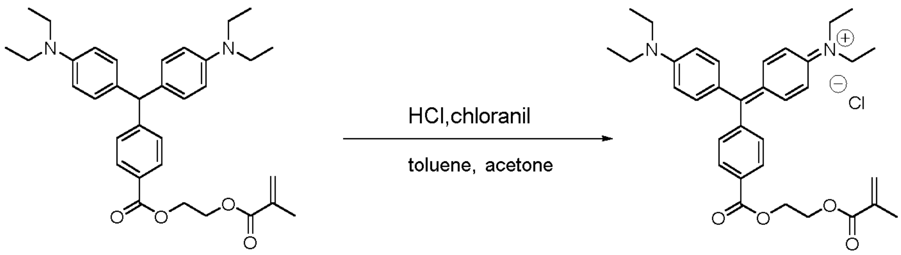


5 7

Example 2

Evaluation of heat resistance of the dye monomer 1 (230°C for 0.5 hour)

Heat resistance of the dye monomer 1 obtained in Example 1 was evaluated as follows.

(1) Synthesis of a polymer not containing a dye.

Into a round-bottom flask equipped with a stirring apparatus, a cooling condenser, a thermometer and a nitrogen introducing unit, 98.5 g of propylene glycol monomethyl ether acetate was added, and heated until inner temperature reached to 90°C, under nitrogen gas flow. Next, a solution mixed with 186.2 g of benzyl methacrylate, 25.6 g of methacrylic acid, and 33.9 g of dimethyl 2,2'-azobis(2-methylpropionate) (a polymerization initiator V-601, produced by Wako Pure Chemical Industries, Ltd.) was dropped into heated propylene glycol monomethyl ether acetate taking 2 hours. After that, the resulting solution was reacted at 90°C for 2 hours. Then, after raising temperature to 100°C, it was reacted for 1 hour. After the reaction, the solution was cooled down to room temperature, and 171.5 g of propylene glycol monomethyl ether acetate was added for dilution to obtain a pale yellow transparent polymer solution. This was referred to as a polymer A. It should be noted that concentration of non-volatile components of the polymer A was 35.9%.

(2) Preparation of a mixed solution of a dye monomer

To prepare a mixed solution B of the dye monomer, 0.08 g of the dye monomer 1, 4.61 g of the polymer A and 2.31 g of propylene glycol monomethyl ether acetate were mixed.

(3) Evaluation of heat resistance

The mixed solution B of the dye monomer was spin coated onto 3 inch glass wafer (Eagle XG, manufactured by Corning Inc.), and then dried for 90 seconds on a hot plate heated at 90°C, to obtain a thin film having a film thickness of 1 $\mu$m. Absorbance ($\lambda$a) at the maximum absorption wavelength of the resulting thin film was measured using a spectrophotometer (Spectrophotometer UV-2550, manufactured by Shimadzu Corp.). After that, the thin film was heated for 30 minutes on the hot plate heated at 230°C, and then absorbance ($\lambda$b) at the maximum absorption wavelength was measured again. From values of $\lambda$a and $\lambda$b, dye residual ratio (%) was determined from the following equation.

$$\text{Dye residual ratio (\%)} = (\lambda b/\lambda a) \times 100$$

Comparative Example 2

Evaluation of heat resistance of the dye monomer 2 (230°C for 0.5 hour)

Heat resistance was evaluated similarly as in Example 2, except for using the dye monomer 2 obtained in Comparative Example 1, instead of the dye monomer 1, as the dye monomer; and using 1-methoxy-2-propanol instead of propylene glycol monomethyl ether acetate.

Comparative Example 3

Evaluation of heat resistance of malachite green (230°C for 0.5 hour)

Heat resistance was evaluated similarly as in Example 2, except for using malachite green oxalate (produced by Wako Pure Chemical Industries, Ltd.), instead of the dye monomer 1, as the dye monomer; and using 1-methoxy-2-propanol instead of propylene glycol monomethyl ether acetate.
Results of Example 2, and Comparative Examples 2 and 3 are shown in Table 1.

TABLE 1

| | Dye residual ratio (%) |
|---|---|
| Example 2 (Dye monomer 1) | 83 |
| Comp. Ex. 2 (Dye monomer 2) | 0 |
| Comp. Ex. 3 (Malachite green) | 0 |

Observation of the glass wafers after heating showed remaining of a green coated film in Example 2, whereas the dye was decomposed and become colorless and transparent in Comparative Example 2. From this result, it has been understood that the dye monomer 1 in Example 2 is a dye having less fading caused by heating. From the result shown by Table 1, it has been understood that the dye monomer 1 in Example 2 showed superior heat resistance, as compared with the dye monomer 2 having a chloride ion as a counter anion, or malachite green which is a general green color dye (a green dye not having a polymerizable group).

Example 3

Synthesis of a dye polymer 1 containing a monomer unit derived from the compound 6

Into a round-bottom flask equipped with a stirring apparatus, a cooling condenser, a thermometer, and a nitrogen introducing unit, 25.6 g of propylene glycol monomethyl ether acetate (produced by Wako Pure Chemical Industries, Ltd.) was charged, and heated until inner temperature reached to 90°C, under nitrogen gas flow. Next, a solution mixed with 2.8 g of the dye monomer 1, 45.9 g of benzyl methacrylate (produced by Wako Pure Chemical Industries, Ltd.), 6.3 g of methacrylic acid (produced by Wako Pure Chemical Industries, Ltd.), and 8.8 g of dimethyl 2,2'-azobis(2-methylpropionate) (a polymerization initiator V-601, produced by Wako Pure Chemical Industries, Ltd.) was dropped into heated propylene glycol monomethyl ether acetate taking 2 hours. After that, the resulting solution was reacted at 90°C for 2 hours, and further at 100°C for 1 hour. After the reaction, the solution was cooled down to room temperature to obtain a dye polymer 1 containing a monomer unit derived from the compound 6 (the compound 6/ benzyl methacrylate/ methacrylic acid = 2.8/45.9/6.3).

Comparative Example 4

Synthesis of a dye polymer 2 containing a monomer unit derived from the compound 7

A dye polymer 2 containing a monomer unit derived from the compound 7 was obtained by carrying out a similar experiment as in Example 3, except for using the dye monomer 2 instead of the dye monomer 1, and using 1-methoxy-2-propanol instead of propylene glycol monomethyl ether acetate.

Example 4

Evaluation of heat resistance of the dye polymer 1 (230°C for 0.5 hour)

Heat resistance of the dye polymer 1 obtained in Example 3 was evaluated as follows.
That is, a dye polymer solution was prepared by mixing 3.0 g of the dye polymer 1 obtained in Example 3, and 4.0 g of propylene glycol monomethyl ether acetate. The prepared polymer solution was spin coated onto 3 inch glass wafer (Eagle XG, manufactured by Corning Inc.), and then dried for 90 seconds on a hot plate heated at 90°C, to obtain a thin film having a film thickness of 1 μm. Absorbance (λa) at the maximum absorption wavelength of the resulting thin film was measured using a spectrophotometer (Spectrophotometer UV-2550, manufactured by Shimadzu Corp.). After

that, the glass wafer was heated for 30 minutes on the hot plate heated at 230°C, and then absorbance (λb) at the maximum absorption wavelength was measured again. From values of λa and λb, dye residual ratio (%) was determined from the following equation. The result thereof is shown in Table 2.

$$\text{Dye residual ratio (\%)} = (\lambda b/\lambda a) \times 100$$

Comparative Example 5

Evaluation of heat resistance of the dye polymer 2 (230°C for 0.5 hour)

Dye residual ratio (%) was determined by a similarly method as in Example 4, except for using the dye polymer 2 obtained in Comparative Example 4, instead of the dye polymer 1 obtained in Example 3; and using 1-methoxy-2-propanol instead of propylene glycol monomethyl ether acetate. The obtained result is shown in Table 2 together with the result in Example 4.

TABLE 2

| | | Dye residual ratio (%) |
|---|---|---|
| Example 4 | Dye polymer 1 | 90 |
| Comp. Ex. 5 | Dye polymer 2 | 0 |

As is clear from the results of Table 1 and Table 2, in the dye monomer 2 having a chloride ion as a counter anion, residual ratio thereof was not enhanced even by polymerizing the dye monomer 2. On the other hand, in the compound of the present invention, it has been understood that the dye residual ratio became higher in using it as the dye polymer containing the monomer unit derived from the monomer, rather than in using it as the dye monomer. From this fact, it has been understood that the heat resistance of the compound of the present invention is enhanced further by polymerizing.

Example 5

Synthesis of a dye polymer 3

Into a 2000 mL round-bottom flask equipped with a stirring apparatus, a cooling condenser, a thermometer, and a nitrogen introducing unit, 105 g of propylene glycol monomethyl ether acetate (produced by Daicel Corp.) was charged, and heated until inner temperature reached to 95°C, under nitrogen gas flow. Next, 15 g of the dye monomer 1 obtained in Example 1, 285 g of methyl methacrylate (produced by Wako Pure Chemical Industries, Ltd.), and 15 g of 2,2'-azobis(methyl 2-methylpropionate) (a polymerization initiator V-601, produced by Wako Pure Chemical Industries, Ltd.) were mixed, and the mixed solution was dropped into the round-bottom flask at 95°C taking 2 hours. After that, the resulting solution was reacted at 95°C for 2 hours. After the reaction, the solution was cooled down to room temperature, and dissolved into 1000 g of ethyl acetate. The mixed solution was charged into 4600 ml of n-hexane, and then a generated precipitate was filtered and dried under reduced pressure to obtain 315 g of a dye polymer 3 containing about 5 parts by weight of the dye monomer 1.

Example 6

Molding of a colored plate containing the dye polymer 3

By using a same direction rotation type twin screw extruder, 0.5 part by weight of the dye polymer 3 obtained in Example 5 and 99.5 parts by weight of a commercially available poly(methyl methacrylate) resin (ACRYPET MD001, produced by Mitsubishi Rayon Co., Ltd.) were melt blended to obtain colored resin pellets. Next, the resulting resin pellets were molded using an electromotive injection molding machine to prepare a colored plate with a size of 150 mm × 150 mm, with a thickness of 2 mm.

Example 7

Elution resistance test of the colored plate containing the dye polymer 3

The colored plate prepared in Example 6 was cut to a size of 40 mm × 30 mm, with a thickness of 2 mm, and then it was immersed in 80 mL of an aqueous ethanol solution, which was prepared by mixing 50 parts of ethanol and 50 parts of ion-exchanged water, and stored in a thermostatic bath at 40°C for 200 hours. During the storage, the aqueous ethanol solution was taken out from the thermostatic bath every 1 hour to measure an optical spectrum thereof using a spectrophotometer (Spectrophotometer UV-2500, manufactured by Shimadzu Corp.). Using absorbance (λa) of the measurement sample at the maximum absorption wavelength and gram absorption coefficient (ε) measured in advance, weight of the dye monomer 1 eluted into the aqueous ethanol solution was calculated, to calculate elution ratio (%), based on weight of the dye monomer 1 contained in the colored plate immersed, by the following equation. The result is shown in Table 3.

$$\text{Elution ratio (\%)} = [(\lambda a \times 0.08/\varepsilon)]/\ (\text{weight of the dye contained in the colored plate})] \times 100$$

(note) Weight of the dye contained in the colored plate = weight of the plate × 0.00025

Table 3

| Example | Colored plate | Elution ratio (%) |
|---|---|---|
| Example 7 | Colored plate containing dye polymer 3 | 0.19 |

As is shown in the Table 3, it has been understood that the polymer of the present invention showed little elution from the colored plate, even in contact with a solvent.

Example 8

Weathering test

The colored plate prepared in Example 6 was cut to a size of 65 mm × 65 mm, with a thickness of 2 mm, and subjected to an accelerated weathering test of a xenon arc lamp type under the following condition, using an apparatus (Ci4000, manufactured by Atlas Material Testing Technology LLC) specified in JIS B7754: 1991.

(1) Test condition

Irradiance: 50 w/$m^2$ (300 to 400 nm)
Filter glass: (inside) borosilicate S-type, (outside) soda lime
Black panel temperature: 63 ± 2°C
Chamber temperature: 38 ± 2°C
Relative humidity: 50 ± 10% RH
Test period: 50 hours
(2) Color measurement condition
Measurement: Reflection measurement (8°:de)
Standard light: $D_{65}$
Measurement hole diameter: φ 5 mm

As for the molded plate before the test and after the test for 50 hours, color difference was measured according

to the L*a*b* color system of JIS Z8730:2009, using a color meter CC-i (manufactured by Suga Test Instruments Co., Ltd.) to calculate ΔL*, Δa* and Δb*, which indicates amount of change of L* value, a* value and b* value, before and after the test; and color difference (ΔE*ab) was determined by the following equation. The obtained results are shown in Table 4.

$$\text{Color difference } (\Delta E^*ab) = [(\Delta L^*)^2 + (\Delta a^*)^2 + (\Delta b^*)^2]^{1/2}$$

Table 4

| Test time | 0 h | | | 50 h | | | Color difference |
|---|---|---|---|---|---|---|---|
| | L* | a* | b* | L* | a* | b* | ΔE*ab |
| Color coordinate | 50.46 | -88.12 | 3.41 | 50.66 | -88.31 | 4.13 | 0.77 |

In the Table 4, when color difference is within 3, it can be judged that there is no color change. Accordingly, it has been understood that the colored plate containing the dye polymer 3 has superior weather resistance.

INDUSTIAL APPLICABILITY

The compound of the present invention or the polymer of the present invention is less fading caused by heating and exerts high heat resistance effect. Accordingly, the colored composition containing the compound or the polymer of the present invention is useful in an application of formation of a colored pixel such as a color filter, as well as applications of printing ink, inkjet ink, paint, and the like; as the one having superior heat resistance effect as compared with the conventional colored compositions.

In addition, the compound or the polymer of the present invention exerts effect of low elution of a dye (a coloring agent), when it is used by mixing in a resist material as a coloring agent. Accordingly, it is useful in applications such as the coloring agent of the resist material, as the superior colored composition having no problem of deterioration in color concentration, color mixture, and the like; as compared with the conventional colored compositions.

**Claims**

**1.** A compound represented by the following general formula (1):

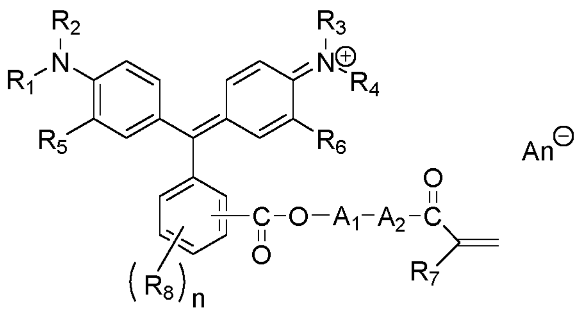

(1)

wherein $R_1$ to $R_4$ each independently represent a hydrogen atom, an alkyl group having 1 to 30 carbon atoms, a hydroxyalkyl group having 1 to 6 carbon atoms, a sulfoalkyl group having 1 to 6 carbon atoms, a carboxyalkyl group having 2 to 7 carbon atoms, a cyanoalkyl group having 2 to 7 carbon atoms, an alkoxyalkyl group having 2 to 6 carbon atoms, a halogenated alkyl group having 1 to 6 carbon atoms, or a phenyl group, a naphthyl group or a benzyl group, having a substituent or not having a substituent; $R_5$ to $R_7$ each independently represent a hydrogen atom or a methyl group; n pieces of $R_8$ each independently represent a halogen atom, an alkyl group having 1 to 21 carbon atoms, an aryl group having 6 to 10 carbon atoms, a hydroxy group, a nitro group, a sulfo group, or an alkoxy group having 1 to 3 carbon atoms; n represents an integer of 0 to 4; $A_1$ represents an alkylene group having 1 to 21 carbon atoms, which has at least one group selected from -O-, -OCO-, -COO- and an arylene group in the chain, an alkylene group having 1 to 21 carbon atoms, which has at least one group selected from -O-, -OCO-, -COO- and an arylene group in the chain, and also has a hydroxy group as a substituent, an alkylene group having

1 to 21 carbon atoms which has a hydroxy group as a substituent, or an alkylene group having 1 to 21 carbon atoms; $A_2$ represents -NH- or -O-; and $An^-$ represents an anion containing an aryl group having an electron-withdrawing substituent, a sulfonyl group having an electron-withdrawing substituent, or a halogenated alkyl group.

**2.** The compound according to claim 1, wherein the electron-withdrawing substituent in $An^-$ is a halogeno group.

**3.** The compound according to claim 1, wherein the electron-withdrawing substituent in $An^-$ is a fluoro group.

**4.** The compound according to claim 1, wherein $An^-$ is a quaternary boron anion.

**5.** The compound according to claim 1, wherein $An^-$ is a tetrakis(perfluorophenyl)borate anion.

**6.** A polymer having a monomer unit derived from the compound represented by the following general formula (1):

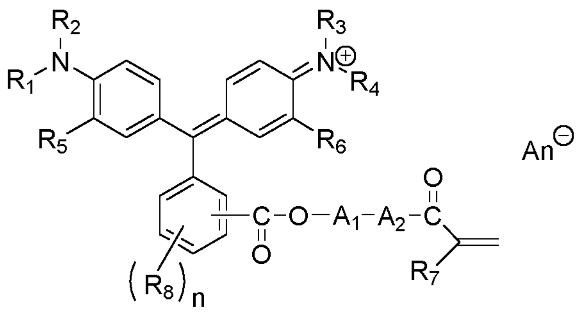

(1)

wherein $R_1$ to $R_4$ each independently represent a hydrogen atom, an alkyl group having 1 to 30 carbon atoms, a hydroxyalkyl group having 1 to 6 carbon atoms, a sulfoalkyl group having 1 to 6 carbon atoms, a carboxyalkyl group having 2 to 7 carbon atoms, a cyanoalkyl group having 2 to 7 carbon atoms, an alkoxyalkyl group having 2 to 6 carbon atoms, a halogenated alkyl group having 1 to 6 carbon atoms, or a phenyl group, a naphthyl group or a benzyl group, having a substituent or not having a substituent; $R_5$ to $R_7$ each independently represent a hydrogen atom or a methyl group; n pieces of $R_8$ each independently represent a halogen atom, an alkyl group having 1 to 21 carbon atoms, an aryl group having 6 to 10 carbon atoms, a hydroxy group, a nitro group, a sulfo group, or an alkoxy group having 1 to 3 carbon atoms; n represents an integer of 0 to 4; $A_1$ represents an alkylene group having 1 to 21 carbon atoms, which has at least one group selected from -O-, -OCO-, -COO- and an arylene group in the chain, an alkylene group having 1 to 21 carbon atoms, which has at least one group selected from -O-, -OCO-, -COO- and an arylene group in the chain, and also has a hydroxy group as a substituent, an alkylene group having 1 to 21 carbon atoms which has a hydroxy group as a substituent, or an alkylene group having 1 to 21 carbon atoms; $A_2$ represents -NH- or -O-; and $An^-$ represents an anion containing an aryl group having an electron-withdrawing substituent, a sulfonyl group having an electron-withdrawing substituent, or a halogenated alkyl group.

**7.** The polymer according to claim 6, wherein the electron-withdrawing substituent in $An^-$ is a halogeno group.

**8.** The polymer according to claim 6, wherein the electron-withdrawing substituent in $An^-$ is a fluoro group.

**9.** The polymer according to claim 6, wherein $An^-$ is a quaternary boron anion.

**10.** The polymer according to claim 6, wherein $An^-$ is a tetrakis(perfluorophenyl)borate anion.

**11.** The polymer according to claim 6, wherein the polymer is a copolymer.

**12.** The polymer according to claim 11, wherein the copolymer is a copolymer having one to two kinds of monomer units derived from a compound represented by the following general formula (2), the general formula (3), the general formula (4) or the general formula (5), and the monomer unit derived from the compound represented by the general formula (1), as configuration components:

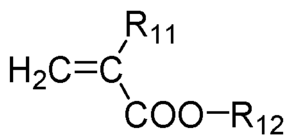

(2)

wherein $R_{11}$ represents a hydrogen atom or a methyl group; $R_{12}$ represents a hydrogen atom, an alkyl group having 1 to 18 carbon atoms, a hydroxyalkyl group having 1 to 10 carbon atoms, an aryl group having 6 to 10 carbon atoms, an arylalkyl group having 7 to 13 carbon atoms, an alkoxyalkyl group having 2 to 9 carbon atoms, an alkoxyalkoxyalkyl group having 3 to 9 carbon atoms, an aryloxyalkyl group having 7 to 13 carbon atoms, a morpholinoalkyl group having 5 to 7 carbon atoms, a trialkylsilyl group having 3 to 9 carbon atoms, an alicyclic hydrocarbon group having 6 to 12 carbon atoms which has an oxygen atom or no oxygen atom, a dialkylaminoalkyl group having 3 to 9 carbon atoms, a fluoroalkyl group having 1 to 18 carbon atoms, an N-alkylenephthalimide group having 9 to 14 carbon atoms, a group represented by the following general formula (2-1):

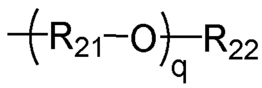

(2-1)

wherein $R_{21}$ represents an alkylene group having 1 to 3 carbon atoms, which has a hydroxy group as a substituent or no substituent; $R_{22}$ represents a phenyl group, which has a hydroxy group as a substituent or no substituent, or an alkyl group having 1 to 3 carbon atoms; and q represents an integer of 1 to 3,
a group represented by the following general formula (2-2):

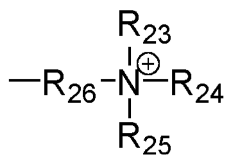

(2-2)

wherein $R_{23}$ to $R_{25}$ represent an alkyl group having 1 to 3 carbon atoms; $R_{26}$ represents an alkylene group having 1 to 3 carbon atoms,
or a group represented by the following general formula (2-3):

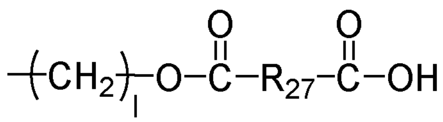

(2-3)

wherein 1 represents an integer of 1 to 6; and $R_{27}$ represents a phenylene group or a cyclohexylene group;

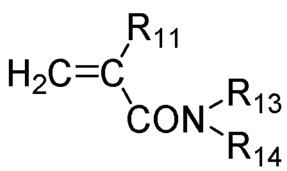

(3)

wherein $R_{11}$ is the same as described above; $R_{13}$ represents a hydrogen atom, or an alkyl group having 1 to 3 carbon atoms; $R_{14}$ represents a hydrogen atom, an alkyl group having 1 to 3 carbon atoms, a dialkylaminoalkyl group having 3 to 9 carbon atoms, or a hydroxyalkyl group having 1 to 6 carbon atoms; and $R_{13}$ and $R_{14}$ may form a morpholino group together with a nitrogen atom adjacent thereto;

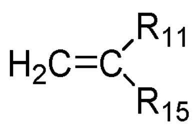

(4)

wherein $R_{15}$ represents a phenyl group or a pyrrolidino group; and $R_{11}$ is the same as described above;

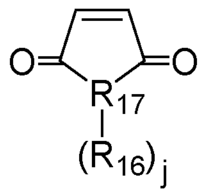

(5)

wherein $R_{17}$ represents a nitrogen atom or an oxygen atom; represents 0 when $R_{17}$ is an oxygen atom, and 1 when $R_{17}$ is a nitrogen atom; $R_{16}$ represents a hydrogen atom, an alkyl group having 1 to 20 carbon atoms, a hydroxyalkyl group having 1 to 10 carbon atoms, a halogenated alkyl group having 1 to 10 carbon atoms, an alkylcycloalkyl group having 6 to 10 carbon atoms, a halogenated cycloalkyl group having 6 to 7 carbon atoms, an aryl group having 6 to 10 carbon atoms, an aryl group having 6 to 10 carbon atoms, which has an alkyl group having 1 to 6 carbon atoms as a substituent, or a halogenated aryl group having 6 to 10 carbon atoms.

**13.** A colored composition comprising the compound according to claim 1 or the polymer according to claim 6.

**14.** A colored composition for a color filter comprising the compound according to claim 1 or the polymer according to claim 6.

**Patentansprüche**

**1.** Eine Verbindung, die durch die folgende allgemeine Formel (1) dargestellt wird:

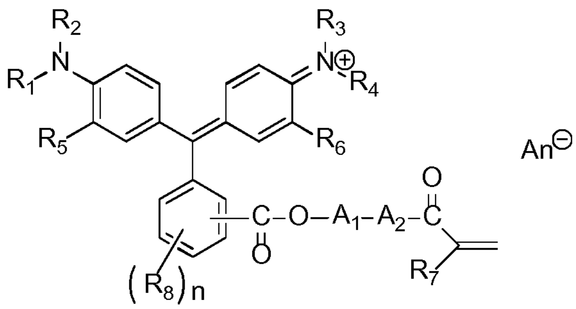

(1)

wobei $R_1$ bis $R_4$ jeweils unabhängig voneinander ein Wasserstoffatom, eine Alkylgruppe mit 1 bis 30 Kohlenstoffatomen, eine Hydroxyalkylgruppe mit 1 bis 6 Kohlenstoffatomen, eine Sulfoalkylgruppe mit 1 bis 6 Kohlenstoffatomen, eine Carboxyalkylgruppe mit 2 bis 7 Kohlenstoffatomen, eine Cyanoalkylgruppe mit 2 bis 7 Kohlenstoffatomen, eine Alkoxyalkylgruppe mit 2 bis 6 Kohlenstoffatomen, eine halogenierte Alkylgruppe mit 1 bis 6 Kohlenstoffatomen oder eine Phenylgruppe, eine Naphthylgruppe oder eine Benzylgruppe mit einem Substituenten oder ohne einen Substituenten darstellen; $R_5$ bis $R_7$ stellen jeweils unabhängig voneinander ein Wasserstoffatom oder eine Methylgruppe dar; n Reste von $R_8$ stellen jeweils unabhängig voneinander ein Halogenatom, eine Alkylgruppe mit 1 bis 21 Kohlenstoffatomen, eine Arylgruppe mit 6 bis 10 Kohlenstoffatomen, eine Hydroxygruppe, eine Nitrogruppe, eine Sulfogruppe oder eine Alkoxygruppe mit 1 bis 3 Kohlenstoffatomen dar; n stellt eine ganze Zahl von 0 bis 4 dar; $A_1$ stellt eine Alkylengruppe mit 1 bis 21 Kohlenstoffatomen, die mindestens eine Gruppe ausgewählt aus -O-, -OCO-, -COO- und einer Arylengruppe in der Kette aufweist, eine Alkylengruppe mit 1 bis 21 Kohlenstoffatomen, die mindestens eine Gruppe ausgewählt aus -O-, -OCO-, -COO- und einer Arylengruppe in der Kette aufweist, und auch eine Hydroxygruppe als einen Substituenten aufweist, eine Alkylengruppe mit 1 bis 21 Kohlenstoffatomen, die eine Hydroxygruppe als einen Substituenten aufweist, oder eine Alkylengruppe mit 1 bis 21 Kohlenstoffatomen, dar; $A_2$ steht für -NH- oder -O-; und $An^-$ stellt ein Anion dar, das eine Arylgruppe mit einem elektronenziehenden Substituenten, eine Sulfonylgruppe mit einem elektronenziehenden Substituenten oder eine halogenierte Alkylgruppe enthält.

**2.** Die Verbindung nach Anspruch 1, wobei der elektronenziehende Substituent in $An^-$ eine Halogengruppe ist.

**3.** Die Verbindung nach Anspruch 1, wobei der elektronenziehende Substituent in $An^-$ eine Fluorogruppe ist.

**4.** Die Verbindung nach Anspruch 1, wobei $An^-$ ein quartäres Boranion ist.

**5.** Die Verbindung nach einem der Ansprüche 1 bis 4, wobei $An^-$ ein Tetrakis(perfluorophenyl)borat-Anion ist.

**6.** Ein Polymer mit einer Monomereinheit, die von der Verbindung der folgenden allgemeinen Formel (1) abgeleitet ist:

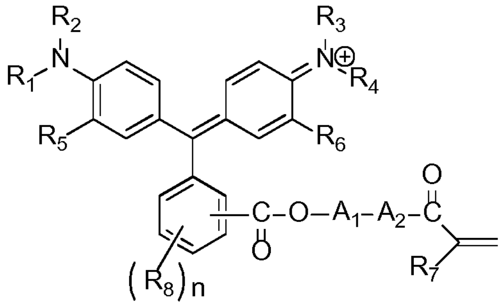

(1)

wobei $R_1$ bis $R_4$ jeweils unabhängig voneinander ein Wasserstoffatom, eine Alkylgruppe mit 1 bis 30 Kohlenstoffatomen, eine Hydroxyalkylgruppe mit 1 bis 6 Kohlenstoffatomen, eine Sulfoalkylgruppe mit 1 bis 6 Kohlenstoffatomen, eine Carboxyalkylgruppe mit 2 bis 7 Kohlenstoffatomen, eine Cyanoalkylgruppe mit 2 bis 7 Kohlenstoffatomen, eine Alkoxyalkylgruppe mit 2 bis 6 Kohlenstoffatomen, eine halogenierte Alkylgruppe mit 1 bis 6 Kohlenstoffatomen oder eine Phenylgruppe, eine Naphthylgruppe oder eine Benzylgruppe mit einem Substituenten oder ohne einen Substituenten darstellen; $R_5$ bis $R_7$ stellen jeweils unabhängig voneinander ein Wasserstoffatom oder eine Methylgruppe dar; n Reste von $R_8$ stellen jeweils unabhängig voneinander ein Halogenatom, eine Alkylgruppe mit 1 bis 21 Kohlenstoffatomen, eine Arylgruppe mit 6 bis 10 Kohlenstoffatomen, eine Hydroxygruppe, eine Nitrogruppe, eine Sulfogruppe oder eine Alkoxygruppe mit 1 bis 10 Kohlenstoffatomen dar. 3 Kohlenstoffatome; n stellt eine ganze Zahl von 0 bis 4 dar; $A_1$ stellt eine Alkylengruppe mit 1 bis 21 Kohlenstoffatomen, die mindestens eine Gruppe ausgewählt aus -O-, -OCO-, -COO- und einer Arylengruppe in der Kette aufweist, eine Alkylengruppe mit 1 bis 21 Kohlenstoffatomen, die mindestens eine Gruppe, ausgewählt aus -O-, -OCO-, -COO- und einer Arylengruppe in der Kette aufweist, und auch eine Hydroxygruppe als einen Substituenten aufweist, eine Alkylengruppe mit 1 bis 21 Kohlenstoffatomen, die eine Hydroxygruppe als einen Substituenten aufweist, oder eine Alkylengruppe mit 1 bis 21 Kohlenstoffatomen, dar; $A_2$ steht für -NH- oder -O-; und $An^-$ stellt ein Anion dar, das eine Arylgruppe mit einem elektronenziehenden Substituenten, eine Sulfonylgruppe mit einem elektronenziehenden Substituenten oder eine halogenierte Alkylgruppe enthält.

**7.** Das Polymer nach Anspruch 6, wobei der elektronenziehende Substituent in $An^-$ eine Halogengruppe ist.

**8.** Das Polymer nach Anspruch 6, wobei der elektronenziehende Substituent in $An^-$ eine Fluorogruppe ist.

**9.** Das Polymer nach Anspruch 6, wobei $An^-$ ein quartäres Boranion ist.

**10.** Das Polymer nach Anspruch 6, wobei $An^-$ ein Tetrakis(perfluorophenyl)borat-Anion ist.

**11.** Das Polymer nach Anspruch 6, wobei das Polymer ein Copolymer ist.

**12.** Das Polymer nach Anspruch 11, wobei das Copolymer ein Copolymer mit ein bis zwei Arten von Monomereinheiten ist, die abgeleitet sind von einer Verbindung der folgenden allgemeinen Formel (2), der allgemeinen Formel (3), der allgemeinen Formel (4) oder der allgemeinen Formel (5), und die Monomereinheit von der Verbindung der allgemeinen Formel (1) abgeleitet ist, als Konfigurationskomponenten:

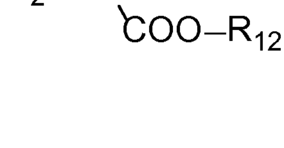

(2)

wobei $R_{11}$ ein Wasserstoffatom oder eine Methylgruppe darstellt; $R_{12}$ ein Wasserstoffatom, eine Alkylgruppe mit 1 bis 18 Kohlenstoffatomen, eine Hydroxyalkylgruppe mit 1 bis 10 Kohlenstoffatomen, eine Arylgruppe mit 6 bis 10 Kohlenstoffatomen, eine Arylalkylgruppe mit 7 bis 13 Kohlenstoffatomen, eine Alkoxyalkylgruppe mit 2 bis 9 Kohlenstoffatome, eine Alkoxyalkoxyalkylgruppe mit 3 bis 9 Kohlenstoffatomen, eine Aryloxyalkylgruppe mit 7 bis 13 Kohlenstoffatomen, eine Morpholinoalkylgruppe mit 5 bis 7 Kohlenstoffatomen, eine Trialkylsilylgruppe mit 3 bis 9 Kohlenstoffatomen, eine alicyclische Kohlenwasserstoffgruppe mit 6 bis 12 Kohlenstoffatomen, die ein Sauerstoffatom oder kein Sauerstoffatom aufweist, eine Dialkylaminoalkylgruppe mit 3 bis 9 Kohlenstoffatomen, eine Fluoroalkylgruppe mit 1 bis 18 Kohlenstoffatomen, eine N-Alkylenphthalimidgruppe mit 9 bis 14 Kohlenstoffatomen, eine Gruppe, die durch die folgende allgemeine Formel (2-1) dargestellt wird:

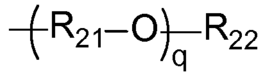 (2-1)

wobei $R_{21}$ eine Alkylengruppe mit 1 bis 3 Kohlenstoffatomen darstellt, die eine Hydroxygruppe als Substituenten oder keinen Substituenten aufweist; $R_{22}$ stellt eine Phenylgruppe dar, die eine Hydroxygruppe als einen Substituenten oder keinen Substituenten aufweist, oder eine Alkylgruppe mit 1 bis 3 Kohlenstoffatomen; und q stellt eine ganze Zahl von 1 bis 3 dar,
eine Gruppe, die durch die folgende allgemeine Formel (2-2) dargestellt wird:

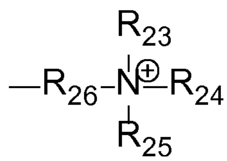 (2-2)

wobei $R_{23}$ bis $R_{25}$ eine Alkylgruppe mit 1 bis 3 Kohlenstoffatomen darstellen; $R_{26}$ eine Alkylengruppe mit 1 bis 3 Kohlenstoffatomen darstellt,
oder eine Gruppe, die durch die folgende allgemeine Formel (2-3) dargestellt wird:

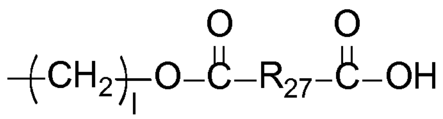 (2-3)

wobei 1 eine ganze Zahl von 1 bis 6 darstellt; und $R_{27}$ eine Phenylengruppe oder eine Cyclohexylengruppe darstellt;

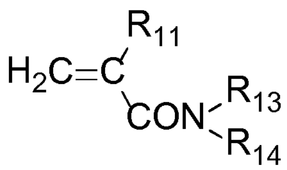 (3)

wobei $R_{11}$ der gleiche Rest ist wie oben beschrieben; $R_{13}$ stellt ein Wasserstoffatom oder eine Alkylgruppe mit 1 bis 3 Kohlenstoffatomen dar; $R_{14}$ stellt ein Wasserstoffatom, eine Alkylgruppe mit 1 bis 3 Kohlenstoffatomen, eine Dialkylaminoalkylgruppe mit 3 bis 9 Kohlenstoffatomen oder eine Hydroxyalkylgruppe mit 1 bis 6 Kohlenstoffatomen dar; und $R_{13}$ sowie $R_{14}$ können zusammen mit einem dazu benachbarten Stickstoffatom eine Morpholinogruppe bilden;

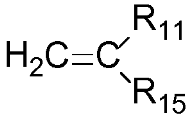 (4)

wobei $R_{15}$ eine Phenylgruppe oder eine Pyrrolidinogruppe darstellt; und $R_{11}$ der gleiche Rest ist wie oben beschrieben;

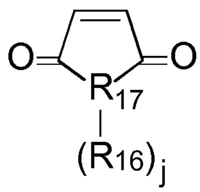

(5)

wobei $R_{17}$ ein Stickstoffatom oder ein Sauerstoffatom darstellt; j steht für 0, wenn $R_{17}$ ein Sauerstoffatom ist, und 1, wenn $R_{17}$ ein Stickstoffatom ist; $R_{16}$ stellt ein Wasserstoffatom, eine Alkylgruppe mit 1 bis 20 Kohlenstoffatomen, eine Hydroxyalkylgruppe mit 1 bis 10 Kohlenstoffatomen, eine halogenierte Alkylgruppe mit 1 bis 10 Kohlenstoffatomen, eine Alkylcycloalkylgruppe mit 6 bis 10 Kohlenstoffatomen, eine halogenierte Cycloalkylgruppe mit 6 bis 7 Kohlenstoffatomen, eine Arylgruppe mit 6 bis 10 Kohlenstoffatomen, eine Arylgruppe mit 6 bis 10 Kohlenstoffatomen, die eine Alkylgruppe mit 1 bis 6 Kohlenstoffatomen als einen Substituenten aufweist, oder eine halogenierte Arylgruppe mit 6 bis 10 Kohlenstoffatomen dar.

**13.** Eine Farbzusammensetzung umfassend die Verbindung nach Anspruch 1 oder das Polymer nach Anspruch 6.

**14.** Eine Farbzusammensetzung für einen Farbfilter, umfassend die Verbindung nach Anspruch 1 oder das Polymer nach Anspruch 6.

**Revendications**

**1.** Un composé représenté par la formule générale (1) suivante:

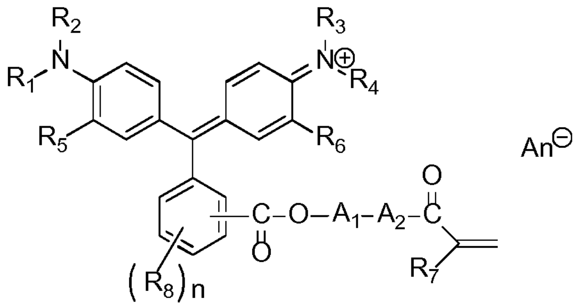

(1)

dans lequel $R_1$ à $R_4$ représentent chacun indépendamment un atome d'hydrogène, un groupe alkyle ayant 1 à 30 atomes de carbone, un groupe hydroxyalkyle ayant 1 à 6 atomes de carbone, un groupe sulfoalkyle ayant 1 à 6 atomes de carbone, un groupe carboxyalkyle ayant 2 à 7 atomes de carbone un groupe cyanoalkyle ayant 2 à 7 atomes de carbone, un groupe alcoxyalkyle ayant 2 à 6 atomes de carbone, un groupe alkyle halogéné ayant 1 à 6 atomes de carbone, ou un groupe phényle, un groupe naphtyle ou un groupe benzyle, ayant un substituant ou non avoir un substituant; $R_5$ à $R_7$ représentent chacun indépendamment un atome d'hydrogène ou un groupe méthyle; n parties de $R_8$ représentent chacune indépendamment un atome d'halogène, un groupe alkyle ayant 1 à 21 atomes de carbone, un groupe aryle ayant 6 à 10 atomes de carbone, un groupe hydroxy, un groupe nitro, un groupe sulfo ou un groupe alcoxy ayant 1 à 3 atomes de carbone; n représente un nombre entier de 0 à 4; $A_1$ représente un groupe alkylène ayant de 1 à 21 atomes de carbone, qui a au moins un groupe choisi parmi -O-, -OCO-, -COO- et un groupe arylène dans la chaîne, un groupe alkylène ayant de 1 à 21 atomes de carbone, a au moins un groupe choisi parmi -O-, -OCO-, -COO- et un groupe arylène dans la chaîne, et a également un groupe hydroxy en tant que substituant, un groupe alkylène ayant de 1 à 21 atomes de carbone qui a un groupe hydroxy en tant que substituant, ou un groupe alkylène ayant de 1 à 21 atomes de carbone; $A_2$ représente -NH- ou -O-; et An- représente un anion contenant un groupe aryle ayant un substituant électroattracteur, un groupe sulfonyle ayant un substituant électroattracteur, ou un groupe alkyle halogéné.

**2.** Le composé selon la revendication 1, dans lequel le substituant électroattracteur dans $An^-$ est un groupe halogéno.

**3.** Le composé selon la revendication 1, dans lequel le substituant électroattracteur dans $An^-$ est un groupe fluoro.

4. Le composé selon la revendication 1, dans lequel $An^-$ est un anion de bore quaternaire.

5. Le composé selon la revendication 1, dans lequel $An^-$ est un anion tétrakis (perfluorophényl) borate.

6. Un polymère ayant un motif monomère dérivé du composé représenté par la formule générale (1) suivante:

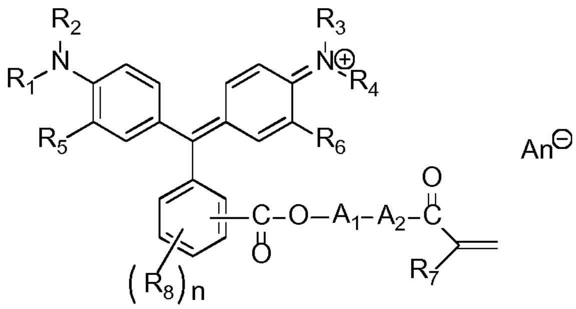

(1)

dans lequel $R_1$ à $R_4$ représentent chacun indépendamment un atome d'hydrogène, un groupe alkyle ayant 1 à 30 atomes de carbone, un groupe hydroxyalkyle ayant 1 à 6 atomes de carbone, un groupe sulfoalkyle ayant 1 à 6 atomes de carbone, un groupe carboxyalkyle ayant 2 à 7 atomes de carbone un groupe cyanoalkyle ayant 2 à 7 atomes de carbone, un groupe alcoxyalkyle ayant 2 à 6 atomes de carbone, un groupe alkyle halogéné ayant 1 à 6 atomes de carbone, ou un groupe phényle, un groupe naphtyle ou un groupe benzyle, ayant un substituant ou non avoir un substituant; $R_5$ à $R_7$ représentent chacun indépendamment un atome d'hydrogène ou un groupe méthyle; n parties de $R_8$ représentent chacune indépendamment un atome d'halogène, un groupe alkyle ayant 1 à 21 atomes de carbone, un groupe aryle ayant 6 à 10 atomes de carbone, un groupe hydroxy, un groupe nitro, un groupe sulfo ou un groupe alcoxy ayant 1 à 3 atomes de carbone; n représente un nombre entier de 0 à 4; $A_1$ représente un groupe alkylène ayant de 1 à 21 atomes de carbone, qui a au moins un groupe choisi parmi -O-, -OCO-, -COO- et un groupe arylène dans la chaîne, un groupe alkylène ayant de 1 à 21 atomes de carbone, a au moins un groupe choisi parmi -O-, -OCO-, -COO- et un groupe arylène dans la chaîne, et a également un groupe hydroxy en tant que substituant, un groupe alkylène ayant de 1 à 21 atomes de carbone qui a un groupe hydroxy en tant que substituant, ou un groupe alkylène ayant de 1 à 21 atomes de carbone; $A_2$ représente -NH- ou -O-; et $An^-$ représente un anion contenant un groupe aryle ayant un substituant électroattracteur, un groupe sulfonyle ayant un substituant électroattracteur, ou un groupe alkyle halogéné.

7. Le polymère selon la revendication 6, dans lequel le substituant électroattracteur dans $An^-$ est un groupe halogéno.

8. Le polymère selon la revendication 6, dans lequel le substituant électroattracteur dans $An^-$ est un groupe fluoro.

9. Le polymère selon la revendication 6, dans lequel $An^-$ est un anion de bore quaternaire.

10. Le polymère selon la revendication 6, dans lequel $An^-$ est un anion tétrakis (perfluorophényl) borate.

11. Le polymère selon la revendication 6, dans lequel le polymère est un copolymère.

12. Le polymère selon la revendication 11, dans lequel le copolymère est un copolymère ayant un à deux types de motifs monomères dérivés d'un composé représenté par la formule générale suivante (2), la formule générale (3), la formule générale (4) ou la formule générale (5), et le motif monomère dérivé du composé représenté par la formule générale (1), en tant que composants de configuration:

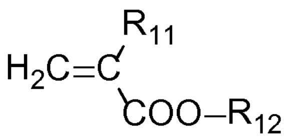

(2)

dans lequel $R_{11}$ représente un atome d'hydrogène ou un groupe méthyle; $R_{12}$ représente un atome d'hydrogène, un groupe alkyle ayant 1 à 18 atomes de carbone, un groupe hydroxyalkyle ayant 1 à 10 atomes de carbone, un groupe aryle ayant 6 à 10 atomes de carbone, un groupe arylalkyle ayant 7 à 13 atomes de carbone, 2 à 9 atomes

de carbone, un groupe alcoxyalcoxyalkyle ayant 3 à 9 atomes de carbone, un groupe aryloxyalkyle ayant 7 à 13 atomes de carbone, un groupe morpholinoalkyle ayant 5 à 7 atomes de carbone, un groupe trialkylsilyle ayant 3 à 9 atomes de carbone, un groupe hydrocarboné alicyclique ayant de 6 à 12 atomes de carbone ayant un atome d'oxygène ou pas d'oxygène, un groupe dialkylaminoalkyle ayant de 3 à 9 atomes de carbone, un groupe fluoroalkyle ayant de 1 à 18 atomes de carbone, un groupe N-alkylènephtalimide ayant de 9 à 14 atomes de carbone, un groupe représenté par la formule générale suivante (2-1):

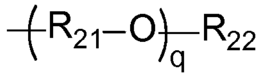
(2-1)

dans lequel $R_{21}$ représente un groupe alkylène ayant de 1 à 3 atomes de carbone, qui a un groupe hydroxy en tant que substituant ou pas de substituant; $R_{22}$ représente un groupe phényle, qui a un groupe hydroxy en tant que substituant ou pas de substituant, ou un groupe alkyle ayant 1 à 3 atomes de carbone; et q représente un nombre entier de 1 à 3,
un groupe représenté par la formule générale suivante (2-2):

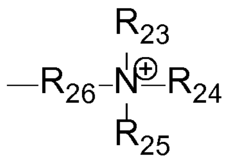
(2-2)

dans lequel $R_{23}$ à $R_{25}$ représentent un groupe alkyle ayant 1 à 3 atomes de carbone; $R_{26}$ représente un groupe alkylène ayant 1 à 3 atomes de carbone,
ou un groupe représenté par la formule générale suivante (2-3):

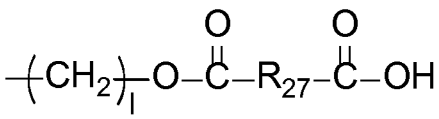
(2-3)

dans lequel 1 représente un nombre entier de 1 à 6; et $R_{27}$ représente un groupe phénylène ou un groupe cyclohexylène;

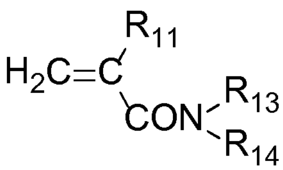
(3)

dans lequel $R_{11}$ est le même que décrit ci-dessus; $R_{13}$ représente un atome d'hydrogène, ou un groupe alkyle ayant 1 à 3 atomes de carbone; $R_{14}$ représente un atome d'hydrogène, un groupe alkyle ayant 1 à 3 atomes de carbone, un groupe dialkylaminoalkyle ayant 3 à 9 atomes de carbone, ou un groupe hydroxyalkyle ayant 1 à 6 atomes de carbone; et $R_{13}$ et $R_{14}$ peuvent former un groupe morpholino avec un atome d'azote adjacent à celui-ci;

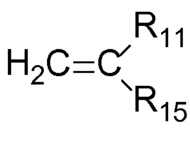
(4)

dans lequel $R_{15}$ représente un groupe phényle ou un groupe pyrrolidino; et $R_{11}$ est le même que décrit ci-dessus;

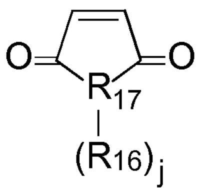

(5)

dans lequel $R_{17}$ représente un atome d'azote ou un atome d'oxygène; j représente 0 lorsque $R_{17}$ est un atome d'oxygène, et 1 lorsque $R_{17}$ est un atome d'azote; $R_{16}$ représente un atome d'hydrogène, un groupe alkyle ayant 1 à 20 atomes de carbone, un groupe hydroxyalkyle ayant 1 à 10 atomes de carbone, un groupe alkyle halogéné ayant 1 à 10 atomes de carbone, un groupe alkylcycloalkyle ayant 6 à 10 atomes de carbone, un groupe cycloalkyle halogéné groupe ayant 6 à 7 atomes de carbone, un groupe aryle ayant 6 à 10 atomes de carbone, un groupe aryle ayant 6 à 10 atomes de carbone, ayant un groupe alkyle ayant 1 à 6 atomes de carbone comme substituant, ou un groupe aryle halogéné ayant 6 à 10 atomes de carbone.

**13.** Une composition colorée comprenant le composé selon la revendication 1 ou le polymère selon la revendication 6.

**14.** Une composition colorée pour un filtre coloré comprenant le composé selon la revendication 1 ou le polymère selon la revendication 6.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010123071 A
- WO 2013108591 A
- WO 2013176383 A1
- JP 2013163804 A
- WO 2013176383 A